# EUROPÄISCHE PATENTANMELDUNG

(11) **EP 3 679 982 A1**
(43) Veröffentlichungstag der Anmeldung: **15.07.2020**
(21) Anmeldenummer: 19172205.7
(22) Anmeldetag: 02.05.2019
(51) Int. Cl.: A61N 1/362

(54) **IMPLANTIERBARE ANORDNUNG ZUR STIMULATION EINES MENSCHLICHEN ODER TIERISCHEN HERZENS**

(30) Priorität: 11.01.2019 DE 102019100610
(71) Anmelder: BIOTRONIK SE & Co. KG, 12359 Berlin (DE)
(72) Erfinder: Dörr, Thomas, 12437 Berlin (DE); Lang, Volker, 12161 Berlin (DE)
(74) Vertreter: Keck, Hans-Georg

(57) **Zusammenfassung**

Die Erfindung betrifft eine implantierbare Anordnung (100) zur Stimulation eines menschlichen oder tierischen Herzens, aufweisend eine erste Stimulationseinheit (103) und eine erste Detektionseinheit (104), wobei die erste Stimulationseinheit (103) einer Stimulation mindestens einer Herzregion eines menschlichen oder tierischen Herzens dient und wobei die erste Detektionseinheit (104) einer Detektion eines elektrischen Signals mindestens einer Herzregion desselben menschlichen oder tierischen Herzens dient. Die Anordnung (100) zeichnet sich dadurch aus, dass sie eine zweite Stimulationseinheit (120) aufweist, die spezifisch dafür ausgebildet und hergerichtet ist, eine Stimulation eines His-Bündels desselben menschlichen oder tierischen Herzens durchzuführen, wobei die zweite Stimulationseinheit (120) für ihre spezifische Ausbildung und Herrichtung eine maximale Stimulationsenergie aufweist, die mindestens 10 % höher als die maximale Stimulationsenergie der ersten Stimulationseinheit (103) ist.

## Beschreibung

Die vorliegende Erfindung betrifft eine implantierbare Anordnung zur Stimulation eines menschlichen oder tierischen Herzens gemäß dem Oberbegriff des Anspruchs 1 sowie ein Computerprogrammprodukt gemäß dem Oberbegriff des Anspruchs 11.

Implantierbare Anordnungen zur Stimulation eines menschlichen oder tierischen Herzens, wie etwa Herzschrittmacher, sind seit langem bekannt. Sie können unterschiedliche Funktionen ausführen. Dabei können verschiedene Stimulationsprogramme von einem entsprechenden Herzschrittmacher durchgeführt werden, um das behandelte Herz wieder in einen normalen Zustand zu bringen.

Aus der US 8,565,880 B2 ist ein Herzschrittmacher bekannt, der zur Stimulation des His-Bündels geeignet ist. Das His-Bündel ist ein Bündel spezifischer Herzmuskelzellen, das Teil des kardialen Erregungsleitungssystems ist. Das His-Bündel liegt distal des atrioventrikulären Knotens in Richtung auf die Herzspitze. Da das His-Bündel eine verhältnismäßig kleine Einheit innerhalb eines menschlichen oder tierischen Herzens darstellt, ist es mitunter schwierig, das His-Bündel mit einer Elektrode korrekt zu treffen und anregen zu können. Das vorgenannte US-Patent beschäftigt sich damit, eine korrekte Erfassung des His-Bündels festzustellen bzw. die Art der Erfassung des His-Bündels in unterschiedliche Kategorien einzuteilen. In Abhängigkeit der bestimmten Kategorie der His-Bündel-Erfassung können dann unterschiedliche Anregungskonfigurationen des Herzschrittmachers vorgegeben werden.

Auch das US-Patent US 8,761,880 B2 beschäftigt sich mit der Feststellung einer His-Bündel-Erfassung. Dazu wird eine Zeit bestimmt, innerhalb derer eine erste kardiale Aktivitätsinformation als Reaktion auf eine His-Bündel-Stimulation erhalten wird. In Abhängigkeit der festgestellten Zeitspanne wird die Güte der His-Bündel-Erfassung ermittelt.

Das US-Patent US 9,168,382 B2 beschäftigt sich ebenfalls mit Verfahren zur Bestimmung einer His-Bündel-Erfassung. Dazu werden kardiale Aktivitätssignale detektiert, die infolge einer angenommenen His-Bündel-Stimulation erzeugt werden. Die erfassten Signale werden dahingehend analysiert, ob sie von leitfähigem Gewebe oder von Myokardgewebe stammen. Nur die Signale, die von leitfähigem Gewebe stammen, werden zur selektiven Verifikation der His-Bündel-Erfassung verwertet.

Somit werden im Stand der Technik bereits einige Verfahren beschrieben, mit denen eine korrekte His-Bündel-Erfassung überprüft werden kann. Allerdings werden zur His-Bündel-Stimulation regelmäßig konventionelle Herzschrittmacher und implantierbare Defibrillatoren eingesetzt. Diese sind jedoch nicht für die Signal- und Zeitgeberkonditionen der His-Bündel-Stimulation angepasst. Insbesondere lassen sich mit diesen konventionellen Systemen regelmäßig keine His-Bündel-Signale mit der erforderlichen Genauigkeit detektieren. Auch weisen die konventionellen Systeme regelmäßig keine spezifischen Stimulationsmodi auf, die an die Anforderungen einer His-Bündel-Stimulation angepasst sind. Darüber hinaus erfordert eine His-Bündel-Stimulation bei den konventionellen Systemen regelmäßig eine Einschränkung existierender Sicherheitsfunktionen und -algorithmen, sodass diese nicht mehr wirksam zur Gewährleistung der Sicherheit der konventionellen Systeme zur Verfügung stehen.

Der vorliegenden Erfindung liegt die Aufgabe zugrunde, die aus dem Stand der Technik bekannten Nachteile konventioneller Herzschrittmacher in Bezug auf eine His-Bündel-Stimulation zu überwinden und ein Gerät bereitzustellen, das sich besonders gut für die His-Bündel-Stimulation eignet.

Diese Aufgabe wird durch eine implantierbare Anordnung zur Stimulation eines menschlichen oder tierischen Herzens mit den Merkmalen des Anspruchs 1 gelöst. Eine derartige Anordnung weist eine erste Stimulationseinheit und eine erste Detektionseinheit auf. Dabei dient die erste Stimulationseinheit einer Stimulation mindestens einer Herzkammer eines menschlichen oder tierischen Herzens. Die erste Detektionseinheit dient der Detektion eines elektrischen Signals mindestens einer Herzkammer desselben menschlichen oder tierischen Herzens. Dabei kann es sich beispielsweise um ein elektrisches Signal handeln, das infolge einer vorherigen Stimulation durch die Stimulationseinheit erzeugt wurde. Es kann sich aber auch um ein elektrisches Signal der Herzkammer handeln, das von der Herzkammer intrinsisch (ohne vorherige externe Stimulation) erzeugt wurde.

Erfindungsgemäß ist vorgesehen, dass die implantierbare Anordnung eine zweite Stimulationseinheit aufweist, die spezifisch dafür ausgebildet und hergerichtet ist, eine Stimulation des His-Bündels desselben menschlichen oder tierischen Herzens durchzuführen. Das heißt, eine Stimulation des His-Bündels des Herzens erfolgt nicht über die konventionell eingesetzte Stimulationseinheit, sondern über eine separate Stimulationseinheit, die ebenfalls in der Anordnung vorgesehen ist. Diese zweite Stimulationseinheit wird dabei hinsichtlich der Anforderungen optimiert, die seitens des His-Bündels für eine Stimulation gestellt werden. Konkret ist die zweite Stimulationseinheit dadurch spezifisch für eine His-Bündel-Stimulation ausgebildet und hergerichtet, dass sie eine maximale Stimulationsenergie aufweist, die mindestens 10 % höher ist als die maximale Stimulationsenergie der ersten Stimulationseinheit. Durch diese höhere maximale Stimulationsenergie kann eine bessere Stimulation des His-Bündels erfolgen. Denn das His-Bündel ist im Vergleich zu anderen kardialen Regionen verhältnismäßig schwierig anzuregen, sodass hierfür regelmäßig eine höhere Energie erforderlich ist. Demgegenüber ist eine derartige höhere Stimulationsenergie bei konventionellen Herzschrittmachern nicht erforderlich oder vorgesehen. Denn diese werden in aller Regel nur zur Stimulation solcher Herzregionen eingesetzt, die sich bereits mit einer geringeren Stimulationsenergie gut stimulieren lassen.

In einer Variante ist die Stimulationsenergie der zweiten Stimulationseinheit mindestens 15 % höher, insbesondere mindestens 20 % höher, insbesondere mindestens 25 % höher, insbesondere mindestens 30 % höher, insbesondere mindestens 35 % höher, insbesondere mindestens 40 % höher, insbesondere mindestens 45 % höher, insbesondere mindestens 50 % höher, insbesondere mindestens 60 % höher, insbesondere mindestens 70 % höher, insbesondere mindestens 80 % höher, insbesondere mindestens 90 % höher, insbesondere mindestens 100 % höher als die maximale Stimulationsenergie der ersten Stimulationseinheit.

In einer Variante ist die maximale Stimulationsenergie der zweiten Stimulationseinheit um einen definierten Prozentsatz höher als die maximale Stimulationsenergie der ersten Stimulationseinheit, der aus einem Intervall aus den vorgenannten Prozentangaben gebildet ist. Das heißt, in dieser Variante ist die maximale Stimulationsenergie der zweiten Stimulationseinheit insbesondere um einen Prozentsatz höher als die maximale Stimulationsenergie der ersten Stimulationseinheit, der zwischen 10 % und 100 %, insbesondere zwischen 15 % und 90 %, insbesondere zwischen 20 % und 80 % etc. liegt.

Um eine Erregung des His-Bündels bzw. eine Aktivität des His-Bündels besonders einfach und sicher detektieren zu können, weist die Anordnung in einer Variante eine zweite Detektionseinheit auf, die spezifisch dafür ausgebildet und hergerichtet ist, ein elektrisches Signal des His-Bündels desselben menschlichen oder tierischen Herzens zu detektieren. Dabei kann die spezifische Ausbildung und Herrichtung der zweiten Detektionseinheit zur Detektion elektrischer Signale des His-Bündels auf unterschiedlichem Wege erreicht werden. So kann die zweite Detektionseinheit eine um mindestens 10 % höhere Sensitivität als die Sensitivität der ersten Detektionseinheit aufweisen. Alternativ oder zusätzlich kann die zweite Detektionseinheit eine um mindestens 10 % größere Detektionsbandbreite als die Detektionsbandbreite der ersten Detektionsvorrichtung aufweisen. Alternativ oder zusätzlich kann die zweite Detektionseinheit eine um mindestens 10 % höhere Abtastrate als die Abtastrate der ersten Detektionsvorrichtung aufweisen. Durch eine derart gegenüber einer konventionellen Detektionseinheit gesteigerten Sensitivität und/oder größeren Detektionsbandbreite und/oder größeren Abtastrate wird die Detektion von Signalen des His-Bündels besonders einfach bzw. überhaupt erst ermöglicht.

In einer Variante ist die Sensitivität der zweiten Detektionseinheit mindestens 15 % höher, insbesondere mindestens 20 % höher, insbesondere mindestens 25 % höher, insbesondere mindestens 30 % höher, insbesondere mindestens 35 % höher, insbesondere mindestens 40 % höher, insbesondere mindestens 45 % höher, insbesondere mindestens 50 % höher, insbesondere mindestens 60 % höher, insbesondere mindestens 70 % höher, insbesondere mindestens 80 % höher, insbesondere mindestens 90 % höher, insbesondere mindestens 100 % höher als die Sensitivität der ersten Detektionseinheit.

In einer Variante ist die Sensitivität der zweiten Detektionseinheit um einen definierten Prozentsatz höher als die Sensitivität der ersten Detektionseinheit, der aus einem Intervall aus den vorgenannten Prozentangaben gebildet ist. Das heißt, in dieser Variante ist die Sensitivität der zweiten Detektionseinheit insbesondere um einen Prozentsatz höher als die Sensitivität der ersten Detektionseinheit, der zwischen 10 % und 100 %, insbesondere zwischen 15 % und 90 %, insbesondere zwischen 20 % und 80 % etc. liegt.

In einer Variante ist die Detektionsbandbreite der zweiten Detektionseinheit mindestens 15 % höher, insbesondere mindestens 20 % höher, insbesondere mindestens 25 % höher, insbesondere mindestens 30 % höher, insbesondere mindestens 35 % höher, insbesondere mindestens 40 % höher, insbesondere mindestens 45 % höher, insbesondere mindestens 50 % höher, insbesondere mindestens 60 % höher, insbesondere mindestens 70 % höher, insbesondere mindestens 80 % höher, insbesondere mindestens 90 % höher, insbesondere mindestens 100 % höher als die Detektionsbandbreite der ersten Detektionseinheit.

In einer Variante ist die Detektionsbandbreite der zweiten Detektionseinheit um einen definierten Prozentsatz höher als die Detektionsbandbreite der ersten Detektionseinheit, der aus einem Intervall aus den vorgenannten Prozentangaben gebildet ist. Das heißt, in dieser Variante ist die Detektionsbandbreite der zweiten Detektionseinheit insbesondere um einen Prozentsatz höher als die Detektionsbandbreite der ersten Detektionseinheit, der zwischen 10 % und 100 %, insbesondere zwischen 15 % und 90 %, insbesondere zwischen 20 % und 80 % etc. liegt.

In einer Variante ist die Abtastrate der zweiten Detektionseinheit mindestens 15 % höher, insbesondere mindestens 20 % höher, insbesondere mindestens 25 % höher, insbesondere mindestens 30 % höher, insbesondere mindestens 35 % höher, insbesondere mindestens 40 % höher, insbesondere mindestens 45 % höher, insbesondere mindestens 50 % höher, insbesondere mindestens 60 % höher, insbesondere mindestens 70 % höher, insbesondere mindestens 80 % höher, insbesondere mindestens 90 % höher, insbesondere mindestens 100 % höher als die Abtastrate der ersten Detektionseinheit.

In einer Variante ist die Abtastrate der zweiten Detektionseinheit um einen definierten Prozentsatz höher als die Abtastrate der ersten Detektionseinheit, der aus einem Intervall aus den vorgenannten Prozentangaben gebildet ist. Das heißt, in dieser Variante ist die Abtastrate der zweiten Detektionseinheit insbesondere um einen Prozentsatz höher als die Abtastrate der ersten Detektionseinheit, der zwischen 10 % und 100 %, insbesondere zwischen 15 % und 90 %, insbesondere zwischen 20 % und 80 % etc. liegt.

In einer Variante weist die Anordnung einen ersten Zeitgeber auf, der dazu dient, Stimulationsimpulse durch die erste Stimulationseinheit zeitlich definiert abzugeben. Darüber hinaus weist die Anordnung in dieser Variante einen zweiten Zeitgeber auf, die dafür vorgesehen und eingerichtet ist, den Zeitpunkt der Abgabe mindestens eines von der zweiten Stimulationseinheit abzugebenden Impulses mit einem Zeitpunkt der Abgabe mindestens eines meiner ersten Stimulationseinheit abzugebenden Impulses abzustimmen. Während die aus dem Stand der Technik bekannten implantierbaren Anordnung zur Stimulation des menschlichen oder tierischen Herzens häufig einen einzigen Zeitgeber aufweisen, mit dem eine Stimulationstherapie in einer für das zu stimulierende Herz sinnvollen zeitlichen Abfolge erfolgen kann, ist das Vorsehen eines zweiten Zeitgebers, der für eine Synchronisation der zweiten Stimulationseinheit mit der ersten Stimulationseinheit sorgt, aus dem Stand der Technik nicht bekannt. Durch diesen zweiten Zeitgeber ist also eine Synchronisation der Stimulation des His-Bündels mit der Stimulation einer anderen Herzregion durch die erste Stimulationseinheit möglich. Auf diese Weise kann besonders einfach ein Synergieeffekt zwischen der His-Bündel-Stimulation und einer konventionellen Stimulation einer anderen Herzregion erreicht werden.

In einer Variante dient der zweite Zeitgeber nicht nur dafür, eine His-Bündel-Stimulation mit einer konventionellen Stimulation einer anderen Herzregionen zu synchronisieren, sondern auch dazu, eine derartige konventionelle Stimulation einer Herzregion mit einer durch die gleiche implantierbare Anordnung durchzuführenden His-Bündel-Stimulation zu synchronisieren. So ist es in dieser Variante vorgesehen, dass der zweite Zeitgeber auch dazu dient, den Zeitpunkt der Abgabe mindestens eines Impulses, der von der ersten Stimulationseinheit abgegeben werden soll, mit einem Zeitpunkt abzustimmen, zu dem mindestens ein Impuls von der zweiten Stimulationseinheit abgegeben werden soll.

In einer Variante weist die Anordnung eine erste Reizschwellentestvorrichtung auf. Mit einer derartigen Reizschwellentestvorrichtung kann eine Reizschwelle des His-Bündels eines menschlichen oder tierischen Herzens, das durch die Vorrichtung stimuliert werden soll, ermittelt werden. Typischerweise steigt eine derartige Reizschwelle nach der Implantation einer entsprechenden Anordnung zunächst an, um in den nachfolgenden Wochen wieder auf ein niedrigeres Niveau abzufallen. Durch eine Reizschwellentestvorrichtung kann ermittelt werden, welche Reizschwelle aktuell überschritten werden muss, um eine ausreichende Stimulation des His-Bündels zu erreichen. Dann kann eine Stimulationsenergie der zweiten Stimulationseinheit in Abhängigkeit der ermittelten Reizschwelle eingestellt werden. Wenn diese Reizschwelle niedriger als kurz nach der Implantation ist, wird auch nur eine niedrigere Stimulationsenergie benötigt. Dadurch reduziert sich der Energieaufwand pro Stimulationsvorgang erheblich, auch wenn ein gleichermaßen guter Stimulationserfolg erzielt wird. Auf diese Weise lässt sich die Lebensdauer der implantierbaren Anordnung verlängern.

Die Reizschwellentestvorrichtung kann beispielsweise eine automatische Reizschwellenüberwachung (Automated Threshold Monitoring, ATM), eine aktive Mitführung der Stimulationsenergie mit jedem Herzschlag (Automated Capture Control, ACC) oder eine automatische Mitführung der Stimulationsenergie ein- oder mehrmals am Tag nach einer Reizschwellenmessung (Automated Threshold Test, ATT) durchführen, um die Reizschwelle des His-Bündels zu ermitteln.

In einer Variante weist die Anordnung nicht nur eine erste Reizschwellentestvorrichtung auf, sondern auch eine zweite Reizschwellentestvorrichtung. Mit einer derartigen zweiten Reizschwellentestvorrichtung kann eine Reizschwelle einer anderen Herzregion (also nicht des His-Bündels) detektiert werden, die durch die erste Stimulationseinheit angeregt werden soll. Dann kann auch für diese Herzregionen die aktuelle Reizschwelle ermittelt werden, um die Stimulationsenergie der ersten Stimulationseinheit in Abhängigkeit dieser ermittelten Reizschwelle einzustellen. Auf diese Weise lässt sich nicht nur die für die His-Bündel-Stimulation benötigte Energie, sondern auch die Energie, die für eine konventionelle Stimulation einer Herzregion benötigt wird, auf das jeweils erforderliche Maß anpassen. Dadurch wird einerseits eine ausreichend hohe Stimulationsenergie gewährleistet, die für einen Stimulationserfolg erforderlich ist. Andererseits wird nur so viel Energie wie nötig zur Stimulation eingesetzt, was - wie vorstehend erläutert - die Lebensdauer der implantierbaren Anordnung verlängert. Auch die zweite Reizschwellentestvorrichtung kann zur Ermittlung der Reizschwelle der betreffenden Herzregionen unterschiedliche Verfahren wie etwa ATM, ACC und ATT ausführen.

In einer Variante weist die Anordnung eine Speichereinheit auf. Diese Speichereinheit umfasst einen Speicherbereich, der ausschließlich zur Speicherung von Daten dient, die von der zweiten Detektionseinheit erfasst wurden und/oder die eine Aktivität der zweiten Stimulationseinheit betreffen. Dabei drückt sich die Aktivität der zweiten Stimulationseinheit insbesondere durch die Art, Frequenz und Anzahl der Stimulationen, die von der zweiten Stimulationseinheit zur Stimulation des His-Bündels abgegeben werden, aus. Dieser Speicherbereich dient somit dazu, alle Vorgänge, die von der implantierbaren Anordnung detektiert bzw. ausgeführt werden und die eine Stimulation des His-Bündels betreffen, zu erfassen. Auf diese Weise lässt sich eine His-Bündel-Stimulation durch die implantierbare Anordnung gesondert von anderen Aktivitäten der implantierbaren Anordnung auslesen und auswerten.

Um eine besonders einfache Möglichkeit zu eröffnen, auf Daten, die innerhalb der implantierbaren Anordnung gespeichert sind, zuzugreifen, weist die Anordnung in einer Variante eine Fernkommunikationseinheit auf. Mittels dieser Fernkommunikationseinheit ist es möglich, Daten, die von der zweiten Detektionseinheit erfasst wurden, und/oder Daten, die eine Aktivität der zweiten Stimulationseinheit betreffen, zu übertragen. Alternativ oder zusätzlich ist es mittels dieser Fernkommunikationseinheit möglich, die zweite Detektionseinheit und/oder die zweite Stimulationseinheit zu überwachen. Alternativ oder zusätzlich ist es mittels der Fernkommunikationseinheit ferner möglich, eine Anpassung einer Stimulation vorzunehmen, die von der zweiten Stimulationseinheit abgegeben werden soll. Das heißt, über die Fernkommunikationseinheit ist ein Fernzugriff auf die zweite Stimulationseinheit möglich. Auf diese Weise lassen sich also aus der Ferne Stimulationsparameter einer durchzuführenden His-Bündel-Stimulation anpassen. Ebenso lassen sich durch die Fernkommunikationseinheit aus der Ferne Daten von der implantierbaren Anordnung abrufen, die eine vorherige His-Bündel-Stimulation widerspiegeln. Schließlich ermöglicht die Fernkommunikationseinheit, eine korrekte Funktionsweise der zweiten Detektionseinheit und/oder der zweiten Stimulationseinheit aus der Ferne zu kontrollieren.

In einer Variante weist die implantierbare Anordnung einen Prozessor und eine Speichereinheit auf. Dabei kann es sich bei der Speichereinheit um die bereits zuvor erläuterte Speichereinheit handeln, die einen spezifischen Speicherbereich aufweist, der für Ereignisse, die die Aktivität bzw. Stimulation des His-Bündels betreffen, reserviert ist. Diese Speichereinheit weist gemäß der vorstehend erläuterten Variante ein computerlesbares Programm auf, das den Prozessor dann, wenn es auf dem Prozessor ausgeführt wird, dazu veranlasst, die nachfolgend erläuterten Schritte auszuführen. Zunächst wird die Anordnung in einen His-Bündel-Stimulationsmodus versetzt, in dem nur eine His-Bündel-Stimulation mittels der zweiten Stimulationseinheit durchgeführt werden kann. Das heißt, dass die erste Stimulationseinheit, die eine konventionelle Stimulation einer anderen Herzregion ermöglichen kann, in diesem His-Bündel-Stimulationsmodus deaktiviert ist. Anschließend wird mittels der zweiten Stimulationseinheit eine His-Bündel-Stimulation in Form von elektrischen Impulsen abgegeben, die zu einer Stimulation des His-Bündels führen soll. Diese Abgabe erfolgt dann, wenn mittels der ersten Detektionseinheit und/oder mittels der zweiten Detektionseinheit ein die Abgabe rechtfertigendes Ereignis detektiert wurde. Wenn also beispielsweise ein Aussetzen des natürlichen Herzschlages bzw. eine unphysiologische Reduzierung der Herzschlagfrequenz erkannt wurde, kann eine Stimulation des His-Bündels mittels der zweiten Stimulationseinheit erfolgen, um wieder einen gewöhnlichen Herzrhythmus herzustellen. Wenn durch die erste Detektionseinheit und/oder die zweite Detektionseinheit anschließend ein gewöhnlicher Herzrhythmus wieder festgestellt werden kann, bedarf es keiner weiteren Stimulation durch die implantierbare Anordnung, insbesondere keiner konventionellen Stimulation einer anderen Region des Herzens mittels der ersten Stimulationseinheit.

Auch in einer weiteren Variante weist die Anordnung einen Prozessor und eine Speichereinheit auf. Dabei kann es sich abermals um dieselbe Speichereinheit handeln, die einen für die His-Bündel-Stimulation reservierten Speicherbereich aufweist und die optional ein Programm aufweist, mit dem die Anordnung in einen His-Bündel-Stimulationsmodus versetzt werden kann. Es ist jedoch auch möglich, dass die nachstehend diskutierte Speichereinheit eine von der vorherigen Speichereinheit unterschiedliche Speichereinheit ist. Die Speichereinheit weist in dieser Variante ein computerlesbares Programm auf, das den Prozessor dazu veranlasst, die nachfolgend erläuterten Schritte auszuführen, wenn es auf dem Prozessor ausgeführt wird. Zunächst wird die Anordnung in einen Sicherheitsmodus versetzt. In diesem Sicherheitsmodus ist sowohl eine Stimulation mindestens einer Herzkammer mittels der ersten Stimulationseinheit als auch eine His-Bündel-Stimulation mittels der zweiten Stimulationseinheit möglich. Das heißt, der Sicherheitsmodus dient dazu, eine grundsätzliche Aktivierbarkeit der ersten Stimulationseinheit und der zweiten Stimulationseinheit zu ermöglichen. Anschließend wird eine Herzkammerstimulation mittels der ersten Stimulationseinheit und/oder eine His-Bündel-Stimulation mittels der zweiten Stimulationseinheit abgegeben. Diese Abgabe erfolgt dann, wenn mittels der ersten Detektionseinheit und/oder mittels der zweiten Detektionseinheit ein die Abgabe rechtfertigendes Ereignis detektiert wurde. Im Sicherheitsmodus wird also nicht nur auf eine His-Bündel-Stimulation gesetzt, sondern gleichzeitig mit der oder zeitlich versetzt zur His-Bündel-Stimulation auch eine konventionelle Stimulation einer beliebigen Herzregion durchgeführt, auf die die erste Stimulationseinheit einwirken kann.

In einer Variante weist die Anordnung einen Markerkanal auf. Über einen derartigen Markerkanal lässt sich ein Elektrokardiogramm (EKG) oder ein intrakardiales Elektrogramm (IEGM) auslesen. Dabei weist das Elektrokardiogramm oder das intrakardiale Elektrogramm mindestens eine Markierung auf, die spezifisch für eine His-Bündel-Stimulation mittels der zweiten Stimulationseinheit und/oder spezifisch für eine Detektion eines Signals des His-Bündels mittels der zweiten Detektionseinheit ist. Es erfolgt also eine spezifische Markierung des auslesbaren Elektrogramms oder des auslesbaren intrakardialen Elektrogramms mit einer His-Bündel-spezifischen Markierung. Mit anderen Worten ausgedrückt, werden His-Bündel-spezifische Marker verwendet, um das EKG bzw. IEGM mit derartigen His-Bündel-spezifischen Markierungen zu versehen. Dabei kann eine Auslesung des EKGs bzw. des IEGMs mittels einer Echtzeit-Telemetrie erfolgen. Alternativ ist es auch möglich, dass das EKG und/oder das IEGM in der Anordnung bzw. in einer Speichereinheit der Anordnung gespeichert werden und anschließend zu einem späteren Zeitpunkt ausgelesen werden können. Dabei ist auch eine Fernübertragung von EKGs bzw. IEGMs möglich. Zu diesem Zweck kann insbesondere eine Fernübertragungseinheit eingesetzt werden. Hierzu eignet sich beispielsweise die weiter oben erläuterte Fernübertragungseinheit.

Um einem Anwender eine Verwendung einer erfindungsgemäßen Anordnung besonders einfach zu machen und um Fehlanwendungen zu vermeiden, weist die Anordnung in einer Variante einen ersten Stimulationsausgang und einen zweiten Stimulationsausgang auf. Der erste Stimulationsausgang dient zum Anschluss einer Stimulationselektrode, die Teil der ersten Stimulationseinheit ist. Der zweite Stimulationsausgang dient zum Anschluss einer zweiten Stimulationselektrode, die Teil der zweiten Stimulationseinheit ist. Mittels der ersten Stimulationselektrode kann eine beliebige Herzregion in einer der beiden Vorhöfe oder Ventrikel (nicht jedoch das His-Bündel) stimuliert werden. Die zweite Stimulationselektrode dient hingegen zur spezifischen Stimulation des His-Bündels des zu behandelnden menschlichen oder tierischen Herzens. Um nun einen Anschluss der ersten Stimulationselektrode an den ersten Stimulationsausgang und insbesondere einen korrekten Anschluss der zweiten Stimulationselektrode an einen zweiten Stimulationsausgang zu ermöglichen, ist zumindest der zweite Stimulationsausgang mit einer spezifischen Markierung versehen, die einem Anwender verdeutlicht, dass der zweite Stimulationsausgang ein Stimulationsausgang ist, der zum Anschluss einer His-Bündel-Stimulationselektrode vorgesehen und eingerichtet ist. Diese spezielle Markierung kann beispielsweise eine Textmarkierung und/oder eine farbliche Markierung des Stimulationsausgangs sein. Dabei kann die Markierung direkt am Stimulationsausgang oder an einem Bereich der implantierbaren Anordnung, die den Stimulationsausgang umgibt bzw. benachbart zu dem Stimulationsausgang angeordnet ist, vorgesehen sein. Beispielsweise kann ein Label oder ein Aufkleber auf der implantierbaren Anordnung vorgesehen sein, der die entsprechende spezifische Ausgestaltung des zweiten Stimulationsausgangs als His-Bündel-Stimulationsausgang kenntlich macht.

Alternativ oder zusätzlich kann darüber hinaus vorgesehen sein, auf der Verpackung der implantierbaren Anordnung eine entsprechende Markierung vorzusehen. Alternativ oder zusätzlich ist vorgesehen, dass in einem Begleitdokument der implantierbaren Anordnung (wie beispielsweise einer Bedienungsanleitung bzw. Anwendungsanleitung) eine entsprechende Markierung vorgesehen ist. Alternativ oder zusätzlich ist eine derartige Markierung auf einer Elektrodenleitung der zweiten Stimulationselektrode vorgesehen. Beispielsweise kann die Elektrodenleitung der zweiten Stimulationselektrode eine spezifische Farbcodierung aufweisen, die mit einer Farbkodierung des zweiten Stimulationsausgangs übereinstimmt. Dann wird einem Anwender besonders einfach visualisiert, welche Stimulationselektrode in welchen Stimulationsausgang der implantierbaren Anordnung einzustecken ist.

Alternativ oder zusätzlich kann eine Markierung, dass der zweite Stimulationsausgang als His-Bündel-Stimulationsausgang konfiguriert ist, auch auf einem Programmiergerät vorgesehen sein, dass zur Programmierung der implantierbaren Anordnung eingesetzt wird. Alternativ oder zusätzlich kann eine derartige Markierung auch in einem Fernüberwachungssystem vorgesehen sein. Beispielsweise kann auf einer grafischen Benutzeroberfläche (GUI) des Fernüberwachungssystems eine entsprechende Markierung vorhanden sein, die einem Benutzer visualisiert, welcher der vorhandenen Stimulationsausgänge ein konventioneller Stimulationsausgang ist und welcher Ausgang ein His-Bündel-Stimulationsausgang ist. Dabei genügt typischerweise die Markierung des His-Bündel-Stimulationsausgangs, da nicht markierte Stimulationsausgänge von einem Benutzer typischerweise als konventionelle Stimulationsausgänge erfasst werden.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn er auf dem Prozessor ausgeführt wird.

Zunächst wird mittels einer ersten Detektionseinheit und/oder einer zweiten Detektionseinheit erfasst, ob in einem menschlichen oder tierischen Herz eine zu behandelnde Herzrhythmusstörung vorliegt. Dabei ist die erste Detektionseinheit zur Detektion eines elektrischen Signals mindestens einer Herzkammer des menschlichen oder tierischen Herzens vorgesehen ist. Die zweite Detektionseinheit ist spezifisch dafür ausgebildet und hergerichtet, ein elektrisches Signal des His-Bündels desselben menschlichen oder tierischen Herzens zu detektieren.

Wenn eine zu behandelnde Herzrhythmusstörung vorliegt, werden anschließend eine atriale oder ventrikuläre Stimulation mittels einer ersten Stimulationseinheit und/oder eine His-Bündel-Stimulation mittels einer zweiten Stimulationseinheit ausgeführt.

Ein Aspekt der vorliegenden Erfindung betrifft ein Verfahren zur Behandlung eines menschlichen oder tierischen Patienten, der eine solche Behandlung benötigt. Dieses Verfahren wird unter Verwendung einer implantierbaren Anordnung gemäß den vorherigen Erläuterungen durchgeführt. Eine derartige Anordnung weist eine erste Stimulationseinheit und eine erste Detektionseinheit auf. Die erste Stimulationseinheit dient zur Stimulation mindestens einer Herzkammer des Herzen des Patienten. Die erste Detektionseinheit dient zur Detektion eines elektrischen Signals mindestens einer Herzkammer des Herzens des Patienten.

Das Verfahren zeichnet sich dadurch aus, dass die Anordnung eine zweite Stimulationseinheit aufweist, die spezifisch dafür ausgebildet und hergerichtet ist, eine Stimulation eines His-Bündels des Herzens des Patienten durchzuführen. Zum Zwecke dieser spezifischen Ausbildung und Herrichtung weist die zweite Stimulationseinheit eine maximale Stimulationsenergie auf, die mindestens 10 % höher als die maximale Stimulationsenergie der ersten Stimulationseinheit ist. Optional kann eine zweite Detektionseinheit vorhanden sein, die spezifisch dafür ausgebildet und hergerichtet ist, ein elektrisches Signal des His-Bündels des Herzens des Patienten zu detektieren.

Gemäß dem Verfahren ist vorgesehen, dass dann, wenn mittels der ersten Detektionseinheit und/oder der zweiten Detektionseinheit ein behandlungspflichtiges Ereignis im Herzen des Patienten detektiert wird, eine Stimulation einer Herzkammer des Patienten mittels der ersten Stimulationseinheit und/oder eine Stimulation des His-Bündels des Patienten mittels der zweiten Stimulationseinheit vorgenommen wird. Dabei führt eine Stimulation des His-Bündels des Herzens des Patienten zu einer gleichzeitigen Stimulation beider Herzkammern. Demgegenüber kann mit der ersten Stimulationseinheit regelmäßig nur eine einzige Herzkammer des Patienten direkt angeregt werden. Somit eignet sich eine Stimulation des His-Bündels insbesondere zur biventrikulären Stimulation und zur ventrikulären Resynchronisation.

Ein Aspekt der vorliegenden Erfindung betrifft eine implantierbare Anordnung zur Stimulation eines menschlichen oder tierischen Herzens mit den nachfolgend erläuterten Merkmalen. Eine derartige Anordnung weist einen Prozessor, eine Speichereinheit, eine Stimulationseinheit und eine Detektionseinheit auf. Die Stimulationseinheit dient zum Stimulieren des His-Bündels eines menschlichen oder tierischen Herzens. Die Detektionseinheit dient zum Detektieren eines elektrischen Signals desselben Herzens.

Erfindungsgemäß zeichnet sich die Anordnung dadurch aus, dass die Speichereinheit ein computerlesbares Programm aufweist, das den Prozessor dazu veranlasst, die nachfolgend erläuterten Schritte auszuführen, wenn es auf dem Prozessor ausgeführt wird.

Zunächst wird eine kardiale Stimulation mittels der Stimulationseinheit ausgeführt.

Anschließend wird mittels der Detektionseinheit ein kardiales elektrisches Signal detektiert. Dieses kardiale elektrische Signal wurde durch eine kardiale Erregung erzeugt, für die die zuvor durchgeführte kardiale Stimulation ursächlich ist.

Das detektierte elektrische Signal wird dann dazu verwendet, den Erregungszustand des durch die kardiale Stimulation stimulierten Herzens zu ermitteln.

Wenn dieser Erregungszustand ermittelt wurde, wird er in einer von mindestens drei Klassen klassifiziert. Dabei umfassen diese mindestens drei Klassen eine erste Klasse, die für einen ersten Erregungszustand vorgesehen ist, und eine zweite Klasse, die für einen zweiten Erregungszustand vorgesehen ist. Dabei unterscheidet sich der zweite Erregungszustand von dem ersten Erregungszustand. Eine dritte Klasse der mindestens drei Klassen ist für eine erfolglose Stimulation ohne detektierbaren Erregungszustand vorgesehen. Somit lassen sich über diese Klassifikation zwei unterschiedliche kardiale Erregungszustände voneinander und zusätzlich gemeinsam von einer erfolglosen Stimulation unterscheiden. Bei einer derartigen erfolglosen Stimulation wird nur ein äußerst schwaches kardiales elektrisches Signal detektiert bzw. ein kardiales elektrisches Signal, das sich nicht oder nur schwach vom Untergrund abhebt. Ein derartiges Signal deutet darauf hin, dass die zuvor durchgeführte Stimulation nicht zu einer kardialen Erregung mit einem ausgeprägten kardialen elektrischen Antwortsignal geführt hat, also erfolglos war.

Nachfolgend erfolgt eine automatische Anpassung zumindest eines Steuerparameters der Anordnung in Abhängigkeit der durchgeführten Klassifikation. Das heißt, die Zuordnung des ermittelten Erregungszustands in eine der mindestens drei Klassen ist ursächlich dafür, wie die Anpassung des mindestens einen Steuerparameters erfolgt. Somit erfolgt eine unmittelbare Berücksichtigung des erzielten Stimulationserfolges durch die implantierbare Anordnung, sodass nachfolgende Stimulationen im Hinblick auf die physiologischen Erfordernisse des zu stimulierenden Herzens optimiert werden.

Sofern sich aus dem ermittelten Erregungszustand ergibt, dass dieser genau dem Erwartungswert entspricht, ist keine gesonderte Anpassung des Steuerparameters erforderlich. Vielmehr würde dann aktiv entschieden werden, dass keine automatische Anpassung vorgenommen werden muss.

Der automatisch anpassbare Steuerparameter ist dabei ausgewählt aus einer His-Bündel-Stimulationsenergie, einem His-Bündel-Stimulationsvektor, eine Betriebsart der Anordnung, mindestens einem Parameter eines Zeitgebers der Anordnung und einem Stimulationssteuerparameter, der für eine kardiale Resynchronisationstherapie geeignet ist. Eine Betriebsart der Anordnung kann beispielsweise eine His-Bündel-Stimulationsbetriebsart und eine konventionelle Stimulationsbetriebsart sein, in der die eingesetzten Algorithmen nicht auf eine Stimulation des His-Bündels angepasst sind. Weitere geeignete Betriebsarten sind eine rechtsventrikuläre Back-up-Stimulation und eine linksventrikuläre Back-up-Stimulation. Die Parameter eines Zeitgebers der Anordnung dienen dazu, die zeitliche Abfolge unterschiedlicher Stimulationen bzw. einzelner Impulse innerhalb einer Stimulation zeitlich zu koordinieren. So sind derartige Zeitgeber typischerweise dafür verantwortlich, ein zeitliches Intervall zwischen einer ersten Stimulation und einer zweiten Stimulation zu definieren.

Die bedarfsgerechte automatische Anpassung des Steuerparameters der Anordnung in Abhängigkeit der zuvor durchgeführten Klassifikation des Erregungszustands des stimulierten Herzens ermöglicht eine Betriebsoptimierung der implantierbaren Anordnung in Bezug auf die His-Bündel-Stimulation.

In einer Variante ist die Stimulationseinheit nicht nur zum Stimulieren des His-Bündels eines menschlichen oder tierischen Herzens geeignet, sondern spezifisch hierfür ausgestaltet und hergerichtet. Dies kann insbesondere über die maximale Stimulationsenergie, die von der Stimulationseinheit abgegeben wird, oder über die Pulsbreite der von der Stimulationseinheit abgegebenen Impulse erreicht werden.

Geeignete Stimulationsamplituden der von der Stimulationseinheit abgegebenen Impulse liegen in einer Variante in einem Bereich von 12 V bis 30 V, insbesondere 15 V bis 25 V, insbesondere 20 V bis 22 V.

Geeignete Pulsbreiten der von der Stimulationseinheit abgegebenen Impulse liegen in einem Bereich zwischen 1 ms und 15 ms, insbesondere zwischen 2 ms und 12 ms, insbesondere zwischen 3 ms und 10 ms, insbesondere zwischen 4 ms und 9 ms, insbesondere zwischen 5 ms und 8 ms.

Die Detektionseinheit kann ebenso entweder besonders geeignet sein, His-Bündel-spezifische Erregungsimpulse des Herzens zu detektieren oder aber spezifisch dafür ausgestaltet und hergerichtet sein. Beispielsweise kann die Detektionseinheit einen Tiefpassfilter aufweisen, der für Signale, die durch eine Erregung des His-Bündels erzeugt werden, besonders geeignet ist. Ein solcher Tiefpassfilter weist in einer Variante eine Grenzfrequenz von mindestens 1 kHz (also 1 kHz oder größer), insbesondere mindestens 500 Hz, insbesondere mindestens 200 Hz und ganz besonders mindestens 100 Hz auf. In einer Variante weist ein solcher Tiefpassfilter eine Grenzfrequenz auf, die in einem Bereich zwischen 100 Hz und 1 kHz, insbesondere zwischen 200 Hz und 500 Hz, insbesondere zwischen 100 Hz und 200 Hz liegt. Signalanteile mit einer Frequenz unterhalb der Grenzfrequenz lässt ein solcher Tiefpassfilter im Wesentlichen ungehindert passieren. Demgegenüber werden Signalanteile mit einer Frequenz, die größer als die Grenzfrequenz ist, abgeschwächt bzw. gedämpft.

Die besondere Eignung der Detektionseinheit zur Detektion von His-Bündel-spezifischen Pulsen bzw. die Ausbildung und Herrichtung der Detektionseinheit für diesen Zweck kann auch durch eine Abtastrate realisiert sein, die in einer Variante mindestens 500 Hz, insbesondere mindestens 1 kHz, insbesondere mindestens 2 kHz, insbesondere mindestens 5 kHz und ganz besonders mindestens 10 kHz beträgt. In einer Variante liegt die Abtastrate der Detektionseinheit in einem Frequenzbereich zwischen 500 Hz und 10 kHz, insbesondere zwischen 1 kHz und 5 kHz, insbesondere zwischen 2 kHz und 4 kHz.

In einer Variante weist die Detektionseinheit eine Empfindlichkeit auf, die in einem Bereich zwischen 0,05 mV und 0,25 mV, insbesondere zwischen 0,1 mV und 0,2 mV liegt. Eine derartige Empfindlichkeit der Detektionseinheit ist besonders gut geeignet, um His-Bündel-spezifische elektrische kardiale Signale detektieren zu können.

Wie bereits erwähnt, handelt es sich bei einer der mindestens drei Klassen um eine Klasse, die für eine erfolglose Stimulation ohne detektierbaren Erregungszustand vorgesehen ist. Die übrigen Klassen der mindestens drei Klassen (also die erste Klasse, die zweite Klasse sowie eine optional vorgesehene weitere Klasse) sind in einer Variante aus den nachfolgend erläuterten Klassen ausgewählt. Eine Klasse betrifft einen Erregungszustand des Herzens, für den ausschließlich eine Stimulation des His-Bündels des stimulierten Herzens erfolgt ist. Das heißt, in diese Klasse fällt ein Erregungszustand, bei dem eine selektive Stimulation des His-Bündels erfolgt ist. Eine weitere Klasse betrifft einen Erregungszustand, für den sowohl eine Stimulation des His-Bündels des Herzens als auch eine Stimulation des ventrikulären Myokards erfolgt ist. Somit betrifft diese Klasse einen Erregungszustand, der auf einer nicht-selektiven Stimulation beruht. Eine Klasse betrifft einen Erregungszustand, für den ausschließlich eine Stimulation des ventrikulären Myokards des stimulierten Herzens erfolgt ist. Das heißt, dieser Erregungszustand beruht auf einer Stimulation, bei der das His-Bündel nicht erfasst wurde. Er kann als rein ventrikulärer Erregungszustand bezeichnet werden. Eine Klasse betrifft einen Erregungszustand, bei dem eine His-Bündel-Stimulation und eine Stimulation linksventrikulärer Leitungsbahnen erfolgt sind. Eine weitere Klasse betrifft einen Erregungszustand, bei dem eine His-Bündel-Stimulation ohne Stimulation linksventrikulärer Leitungsbahnen erfolgt ist. Eine derartige Stimulation wird auch als Rechtsschenkelblock bzw. Rechtsschenkelblockbild (RSB bzw. RBBB) bezeichnet.

Die Klassifikation des Erregungszustandes in eine der vordefinierten Klassen bedeutet automatisch eine Klassifikation der dem Erregungszustand zu Grunde liegenden Stimulation in eine derartige Klasse.

In einer Variante veranlasst das Programm den Prozessor dazu, einmal oder mehrmals täglich einen automatischen Reizschwellentest (ATM) durchzuführen. Im Rahmen eines derartigen Reizschwellentest wird eine Reizschwelle für eine erfolgreiche His-Bündel-Stimulation bestimmt. Dabei wird eine selektive oder nicht selektive His-Bündel-Stimulation als erfolgreich angesehen, während eine Stimulation, bei der das His-Bündel nicht erfasst ist bzw. die lediglich eine ventrikuläre Stimulation zur Folge hat, als nicht erfolgreich angesehen werden.

In einer Variante veranlasst das Programm den Prozessor dazu, jede mittels der Stimulationseinheit ausgeführte kardiale Stimulation zu klassifizieren. Hierfür kann beispielsweise eine aktive Erfassungskontrolle (ACC), wahlweise unter Anwendung eines Beat-to-Beat-Algorithmus, angewendet werden.

In einer Variante ist die Anordnung als Einkanalsimulator ausgestaltet, der nur einen einzigen Anschluss für eine Stimulations- und Detektionselektrode aufweist. Dabei ist die Stimulations- und Detektionselektrode Teil der Stimulationseinheit und der Detektionseinheit. Somit kann sie auch als His-Bündel-Stimulations- und Detektionselektrode oder als His-Bündel-Stimulations- und Detektionskanal bezeichnet werden. Dabei ist die Detektionseinheit nicht nur zur Detektion von His-Bündel-spezifischen kardialen Signalen des zu stimulierenden Herzens vorgesehen und eingerichtet. Vielmehr ist sie auch dafür vorgesehen und eingerichtet, eine intrinsische Erregung eines der beiden Atrien des zu stimulierenden Herzens zu detektieren. Signale, die aus einer derartigen intrinsischen Erregung eines der beiden Atrien resultieren, werden auch als P-Welle bezeichnet. Wenn die Detektionseinheit zur Detektion derartiger atrialer Signale vorgesehen und eingerichtet ist, lässt sich die Stimulation des His-Bündels des zu stimulierenden Herzens durch die Stimulationseinheit besonders leicht in Abhängigkeit dieser intrinsischen atrialen Signale triggern bzw. ausführen.

In einer Variante ist die implantierbare Anordnung als Zweikanalstimulator ausgestaltet. Sie weist dann einen ersten Anschluss für eine His-Bündel-Stimulations- und Detektionselektrode (bzw. für einen His-Bündel-Stimulations- und Detektionskanal) und einen zweiten Anschluss auf, der als atrialer Stimulations- und Detektionskanal oder als ventrikulärer Stimulations- und Detektionskanal zum Anschluss einer entsprechenden atrialen Stimulations- und Detektionselektrode oder einer ventrikulären Stimulations- und Detektionselektrode ausgestaltet sein kann. In solch einer Variante weist die Anordnung neben der Stimulationseinheit und der Detektionseinheit typischerweise eine zweite Stimulationseinheit und eine zweite Detektionseinheit auf, der die atriale oder ventrikuläre Stimulations- und Detektionselektrode dann zugeordnet ist.

In einer Variante ist die implantierbare Anordnung als Dreikanalstimulator ausgestaltet. Ein derartiger Dreikanalstimulator weist einen ersten Anschluss für einen His-Bündel-Stimulations- und Detektionskanal (bzw. für eine His-Bündel-Stimulations- und Detektionselektrode), einen zweiten Anschluss für einen atrialen Stimulations- und Detektionskanal (bzw. für eine atriale Stimulations- und Detektionselektrode) und einen dritten Anschluss für einen ventrikulären Stimulations- und Detektionskanal (bzw. für eine ventrikuläre Stimulations- und Detektionselektrode auf). Mit einem derartigen Dreikanalstimulator können also sowohl His-Bündel-spezifische Stimulationen durchgeführt und His-Bündel-spezifische Signale erfasst werden als auch atriale und ventrikuläre Stimulationen durchgeführt und atriale und ventrikuläre Signale besonders leicht detektiert werden.

In einer Variante ist die implantierbare Anordnung als Vierkanalstimulator ausgestaltet. Ein derartiger Vierkanalstimulator weist einen ersten Anschluss für einen His-Bündel-Stimulations- und Detektionskanal (bzw. für eine His-Bündel-Stimulations- und Detektionselektrode), einen zweiten Anschluss für einen atrialen Stimulations- und Detektionskanal (bzw. für eine atriale Stimulations- und Detektionselektrode), einen dritten Anschluss für einen ersten ventrikulären Stimulations- und Detektionskanal (bzw. eine erste ventrikuläre Stimulations- und Detektionselektrode) und einen vierten Anschluss für einen zweiten ventrikulären Stimulations- und Detektionskanal (bzw. für eine zweite ventrikuläre Stimulations- und Detektionselektrode) auf. Mit einem derartigen Vierkanalstimulator können also beispielsweise beide Ventrikel eines menschlichen oder tierischen Herzens gleichzeitig stimuliert werden. In gleicher Weise können gleichzeitig Signale aus beiden Ventrikeln erfasst werden. Darüber hinaus ist die bereits in Zusammenhang mit den vorherigen Varianten erläuterte His-Bündel-spezifische Stimulation und Detektion sowie eine atriale Stimulation und Detektion möglich.

In einer weiteren Variante ist die implantierbare Anordnung als Fünfkanalstimulator ausgestaltet, der insgesamt fünf Anschlüsse aufweist. Ein erster Anschluss ist für einen His-Bündel-Stimulations- und Detektionskanal (bzw. für eine His-Bündel-Stimulations- und Detektionselektrode) vorgesehen. Ein zweiter Anschluss ist für einen atrialen Stimulations- und Detektionskanal (bzw. für eine atriale Stimulations- und Detektionselektrode) vorgesehen. Ein dritter Anschluss dient zum Anschließen eines ersten ventrikulären Stimulations- und Detektionskanals (bzw. einer ersten ventrikulären Stimulations- und Detektionselektrode). Ein vierter Anschluss ist für einen zweiten ventrikulären Stimulations- und Detektionskanal (bzw. für eine zweite ventrikuläre Stimulations- und Detektionselektrode) vorgesehen. Schließlich ist ein fünfter Anschluss für einen dritten ventrikulären Stimulations- und Detektionskanal (bzw. für eine dritte ventrikuläre Stimulations- und Detektionselektrode) vorgesehen. So kann die implantierbare Anordnung beispielsweise als Stimulator eingesetzt werden, der für die kardiale Resynchronisation (CRT) eingerichtet ist (CRT-Stimulator) und der einen quadropolaren linksventrikulären Kanal aufweist. Alternativ kann die implantierbare Anordnung eine multipolare Stimulation (ein sogenanntes Multipol-Pacing) unter Verwendung eines His-Bündel-spezifischen Stimulationskanals ermöglichen.

In einer Variante weist die implantierbare Anordnung eine Einrichtung zur automatischen Kardioversion und/oder Defibrillation des Herzens auf und ist mit entsprechenden Anschlüssen ausgestattet, um diese Einrichtung mit geeigneten Herzregionen zu verbinden.

In einer Variante weist die Anordnung eine Stimulationserfolgskontrolleinheit auf, die als ein hardwaretechnisch von der Stimulationseinheit und der Detektionseinheit separat ausgeführtes Modul ausgestaltet ist. Dabei übernimmt diese Stimulationserfolgskontrolleinheit zumindest einige Schritte, mit denen eine Erfolgskontrolle einer zuvor ausgeführten kardialen Stimulation durchgeführt wird, insbesondere die Schritte der Ermittlung des Erregungszustands des stimulierten Herzens und der Klassifikation des Erregungszustands eine von mindestens drei Klassen. Eine derartige Stimulationserfolgskontrolleinheit kann dann geeignete Steuersignale an die Stimulationseinheit oder eine Steuereinheit der implantierbaren Anordnung senden, um eine automatische Anpassung mindestens eines Steuerparameters der Anordnung zu erreichen.

Für die His-Bündel-Stimulation sind in der Stimulationseinheit grundsätzlich mindestens zwei Stimulationsreizschwellen von Bedeutung. Die erste dieser Reizschwellen ist die Reizschwelle für die Stimulation des ventrikulären Myokards. Diese ist vergleichbar mit der Gewebereizschwelle konventioneller Stimulationskanäle und hängt insbesondere von der elektrischen Anbindung der entsprechenden Elektrode zum Myokard und von den grundsätzlichen Stimulierbarkeitseigenschaften des entsprechenden Myokards ab. Die zweite Reizschwelle ist die Reizschwelle der eigentlichen His-Bündel-Stimulation. Eine Überschreitung dieser Reizschwelle ist erforderlich, um die His-Bündel-Strukturen selbst ausreichend zu erregen, sodass die Effekte der durchgeführten His-Bündel-Stimulation erreicht werden, insbesondere dass eine Erregungsausbreitung erreicht wird.

Bei konventionellen Anordnungen zur Stimulation eines menschlichen oder tierischen Herzens, wie etwa konventionellen Herzschrittmachern, ist eine Verlaufskontrolle beider Reizschwellen nicht möglich. Vielmehr wird hier stets nur eine einzige Reizschwelle pro Stimulationskanal erfasst und gespeichert. Wenn jedoch nur eine der beiden relevanten Reizschwellen erfasst und gespeichert werden kann, besteht die Gefahr einer Fehlbedienung und einer Fehlinterpretation des abgespeicherten Ergebnisses. Insbesondere dann, wenn unterschiedliche Personen damit betraut werden, die entsprechende Reizschwelle zu identifizieren und zu speichern, besteht eine signifikante Verwechslungsgefahr. Wenn nämlich eine erste Person die erste Reizschwelle (also die Reizschwelle des ventrikulären Myokards) als relevante Reizschwelle identifiziert und speichert, eine andere Person jedoch die zweite Reizschwelle (also die His-Bündel-Reizschwelle) als relevante Reizschwelle identifiziert und speichert, können bei der Verlaufskontrolle der Reizschwelle Fehlinterpretationen die Folge sein. Beispielsweise könnte dann, wenn versehentlich statt der Reizschwelle des ventrikulären Myokards zumindest zeitweise die Reizschwelle der His-Bündel-Stimulation gespeichert und ausgewertet wird, die Fehldiagnose gestellt werden, dass ein genereller Reizschwellenanstieg erfolgt ist, der auf eine schlechtere Stimulierbarkeit hindeutet, wofür beispielsweise nekrotisches Gewebe verantwortlich sein könnte. Tatsächlich hätte sich in einem solchen Fall der Anteil nekrotischen Gewebes im Herzen jedoch gar nicht verändert; es wurde lediglich der "falsche" Reizschwellenwert betrachtet.

In einer Variante veranlasst das Programm den Prozessor daher dazu, mindestens zwei Reizschwellenmesswerte zum selben Zeitpunkt zu bestimmen und in einen Messwertspeicher abzuspeichern. Dabei gibt ein erster Reizschwellenmesswert eine Reizschwelle eines ersten Erregungszustands des stimulierten Herzens an, während ein zweiter Reizschwellenmesswert eine Reizschwelle eines zweiten Erregungszustands des stimulierten Herzens angibt.

Der Messwertspeicher kann ein definierter Speicherbereich der Speichereinheit sein oder auch als separat von der Speichereinheit ausgestaltete Speicher ausgeführt sein. Durch die zeitlich synchrone Erfassung zweier unterschiedlicher Reizschwellenmesswerte eröffnet sich die Möglichkeit, zwei unterschiedliche Reizschwellen zu überwachen und somit unterschiedliche medizinische Rückschlüsse aus diesen überwachten Reizschwellen zu ziehen.

In einer Variante sind der erste Erregungszustand und der zweite Erregungszustands aus den nachfolgend erläuterten Erregungszuständen ausgewählt. So kann es sich um einen Erregungszustand handeln, für den ausschließlich eine Stimulation des His-Bündels des stimulierten Herzens erfolgt ist (selektive Stimulation). Ferner kann der Erregungszustand ein Zustand sein, für den sowohl eine Stimulation des His-Bündels des stimulierten Herzens als auch eine Stimulation des ventrikulären Myokards erfolgt ist (nicht-selektive Stimulation). Schließlich kann der Erregungszustand ein Zustand sein, für den ausschließlich eine Stimulation des ventrikulären Myokards des stimulierten Herzens (ohne Stimulation des His-Bündels) erfolgt ist.

Durch eine derartige Unterteilung der unterschiedlichen Erregungszustände und der Zuordnung der mindestens zwei bestimmten Reizschwellenmesswerte zu einem solchen Erregungszustand ist es also möglich, die Reizschwelle einer His-Bündel-Stimulation von der Reizschwelle einer ventrikulären Myokard-Stimulation zu unterscheiden. Mithin ist es möglich, durch eine im zeitlichen Verlauf eintretende Erhöhung der Reizschwelle einer ventrikulären Myokard-Stimulation beispielsweise eine histologische Veränderung des stimulierten Herzens zu erkennen. Denn wenn die Reizschwelle, die für die ventrikuläre Myokard-Stimulation relevant ist, ansteigt, deutet dies auf eine insgesamt schlechtere Stimulierbarkeit des zu stimulierenden menschlichen oder tierischen Herzens hin, die ihre Ursache in einer Erhöhung des Anteils nekrotischen Gewebes haben kann.

Steigt hingegen im zeitlichen Verlauf die Reizschwelle an, die für die His-Bündel-Stimulation relevant ist, deutet dies nicht auf eine schlechtere Stimulierbarkeit des Herzens insgesamt hin, sondern vielmehr auf eine (gegebenenfalls nur geringfügige) Dislokation der entsprechenden Stimulationselektrode. Somit lassen sich mittels der getrennten Überwachung zweier unterschiedlicher Reizschwellen medizinisch relevante Daten gewinnen, die das Risiko einer Fehldiagnose signifikant reduzieren und somit einen wesentlichen Einfluss auf die Patientensicherheit haben.

Ein Aspekt der vorliegenden Erfindung betrifft daher eine implantierbare Anordnung zur Stimulation eines menschlichen oder tierischen Herzens, bei der eine derartige Erfassung mindestens zweier Reizschwellenmesswerte - einschließlich der vorstehend und nachfolgend erläuterten Varianten dieser Reizschwellenmesswerterfassung - vorgesehen ist, die aber keine Klassifikation eines festgestellten Erregungszustands in eine von mindestens drei vordefinierten Klassen und keine nachfolgende automatische Anpassung eines Steuerparameters der Anordnung in Abhängigkeit der durchgeführten Klassifikation umfasst. Eine derartige Anordnung lässt sich auch wie folgt beschreiben:
Implantierbare Anordnung zur Stimulation eines menschlichen oder tierischen Herzens, aufweisend einen Prozessor, eine Speichereinheit, eine Stimulationseinheit zum Stimulieren eines His-Bündels eines menschlichen oder tierischen Herzens und eine Detektionseinheit zum Detektieren eines elektrischen Signals desselben Herzens, dadurch gekennzeichnet, dass die Speichereinheit ein computerlesbares Programm aufweist, das den Prozessor dazu veranlasst, die folgenden Schritte auszuführen, wenn es auf dem Prozessor ausgeführt wird:
a) Ausführen einer kardialen Stimulation mittels der Stimulationseinheit,
b) Detektieren eines kardialen elektrischen Signals, das durch eine kardiale Erregung infolge der zuvor durchgeführten kardialen Stimulation erzeugt wurde, mittels der Detektionseinheit,
c) Ermittlung eines Erregungszustands eines durch die kardiale Stimulation stimulierten Herzens mittels des elektrischen Signals,
d) Bestimmung zweier Reizschwellenmesswerte zum selben Zeitpunkt und Abspeichern der Reizschwellenmesswerte in einem Messwertspeicher, wobei ein erster Reizschwellenmesswert eine Reizschwelle eines ersten Erregungszustands des stimulierten Herzens und ein zweiter Reizschwellenmesswert eine Reizschwelle eines zweiten Erregungszustands des stimulierten Herzens angibt.

Die vorstehend und nachfolgend erläuterten Varianten und Ausführungsbeispiele beziehen sich sowohl auf die implantierbare Anordnung zur Stimulation eines menschlichen oder tierischen Herzens, bei der eine Klassifikation eines Erregungszustands in eine von mindestens drei Klassen und nachfolgend eine automatische Anpassung mindestens eine Steuerparameters vorgenommen wird, als auch auf solche Varianten, bei denen diese Klassifikation und automatische Anpassung nicht vorgenommen wird. Darüber hinaus sind die nachfolgend erläuterten Varianten auch mit weiteren Aspekten der vorliegend beschriebenen Erfindung kombinierbar.

In einer Variante veranlasst das Programm den Prozessor dazu, einen zeitlichen Verlauf der mindestens zwei Reizschwellenmesswerte abzuspeichern. Durch die Abspeicherung eines derartigen Verlaufs ist eine zeitliche Verlaufskontrolle der Reizschwellenmesswerte besonders einfach möglich. Auf diese Weise lässt sich besonders einfach ein Trend der Reizschwellenmesswerte erkennen, aus dem dann die entsprechenden Rückschlüsse hinsichtlich einer möglichen Veränderung der kardialen Stimulierbarkeit oder einer etwaigen Dislokation einer Stimulationselektrode ableitbar sind.

In einer Variante ist die Anordnung dafür vorgesehen und eingerichtet, für jeden der mindestens zwei Reizschwellenmesswerte einen Warnschwellenwert festzulegen. Dabei ist vorgesehen, dass das Programm den Prozessor dazu veranlasst, einen Warnhinweis abzugeben, wenn zumindest einer der Warnschwellenwerte überschritten wird. Auf diese Weise ist es möglich, einen Nutzer der implantierbaren Anordnung frühzeitig über Abweichungen vom erwarteten Verhalten der Anordnung zu informieren. Dann können frühzeitig Maßnahmen getroffen werden, um eine mögliche Gewebeveränderung des zu stimulierenden menschlichen oder tierischen Herzens aufzuhalten oder rückzubilden oder um eine Relokation einer Elektrode vorzunehmen.

Die Warnschwellenwerte können beispielsweise in der implantierbaren Anordnung selbst, in einem Programmiergerät, das zur Programmierung der implantierbaren Anordnung bzw. zur Programmierung von deren Komponenten eingesetzt wird, oder in einem Fernüberwachungssystem, mit dem mittels Datenfernübertragung auf die implantierbare Anordnung zugegriffen werden kann, hinterlegt sein.

In einer Variante weisen die im Messwertspeicher abgespeicherten Reizschwellenmesswerte jeweils mindestens ein Wertepaar auf, dass einerseits die Reizschwelle in Form einer Amplitude (gemessen in Volt oder einer vergleichbaren Spannungseinheit) und andererseits eine Pulsbreite (gemessen in Millisekunden oder einer anderen Zeiteinheit) betreffen. Typischerweise kann eine Reizschwelle dann als Spannung bei einer bestimmten Pulsbreite angegeben werden. In einer weiteren Variante wird jedem Reizschwellenmesswert zudem ein Zeitpunkt der Messung zugeordnet.

Um die unterschiedlichen Reizschwellen besonders leicht identifizieren zu können, sind diese in einer Variante mit den Bezeichnungen "His-Bündel-selektiv", "His-Bündel-nichtselektiv" und "nur ventrikulär" oder inhaltlich vergleichbaren Bezeichnungen versehen. So lassen sich beispielsweise Darstellungen auf einer grafischen Benutzeroberfläche oder in Ausdrucken entsprechend kennzeichnen, um medizinischem Fachpersonal, das die entsprechenden Daten auswertet, die Arbeit zu erleichtern.

In einer Variante weist die Anordnung eine Datenfernübertragungseinheit auf, mittels der eine Übertragung der gespeicherten Reizschwellenmesswerte beispielsweise an ein Fernüberwachungssystem möglich ist. In einem derartigen Fernüberwachungssystem können beispielsweise Reizschwellenmesswerte zahlreicher unterschiedlicher implantierbarer Anordnung zur Stimulation des Herzens gesammelt und ausgewertet werden. Es ist dann möglich, von einem derartigen zentralen Fernüberwachungssystem Reizschwellenmesswertverläufe in unterschiedlichen implantierbaren Anordnungen zu überwachen und bei Bedarf die Nutzer dieser Anordnungen zu informieren bzw. zur Korrektur auf die Anordnungen zuzugreifen.

Für eine besonders einfache Auswertung werden die Reizschwellenmesswerte in einer Variante zusammen mit einer Differenz zu einem vorgebbaren Reizschwellenwert dargestellt. Dann lässt sich besonders leicht erkennen, ob der aktuell ermittelte Reizschwellenmesswert höher oder niedriger als der vorgegebene bzw. vorgebbare Reizschwellenwert ist.

Um eine einfache Unterscheidung der ermittelten Reizschwellenmesswerte voneinander und eine besonders einfache Zuordnung zu einer Reizschwellenart zu erreichen, ist es in einer Variante vorgesehen, dass ein Anwender den Reizschwellen mindestens eine der folgenden Beschreibungen der gewählten kardialen Stimulationsart zuordnen kann: "niedrige atriale Stimulation", "kombinierte atriale Stimulation und His-Bündel-Stimulation", "His-Bündel-Stimulation", "kombinierte atriale Stimulation, ventrikulär-septale Stimulation und His-Bündel-Stimulation", "kombinierte ventrikulär-septale Stimulation und His-Bündel-Stimulation" und "ventrikuläre Stimulation". Durch die Identifikation derartiger Stimulationsarten mit Klarnamen können unterschiedliche Reizschwellen besonders einfach den jeweiligen Stimulationsarten zugeordnet werden. Auf diese Weise kann eine Verwechslung unterschiedlicher Reizschwellen vermieden werden.

In einer Variante veranlasst das Programm den Prozessor dazu, vor der Ermittlung des Erregungszustands eine Signalqualitätsüberprüfung (nach dem englischen Fachbegriff "signal quality check" auch als SQC abgekürzt) durchzuführen. Mit einer derartigen Signalqualitätsüberprüfung lässt sich feststellen, ob die zur Ermittlung des Erregungszustands vorgesehenen Signale zur Auswertung geeignet sind. Diese Eignung kann sich beispielsweise aus der absoluten Signalintensität, aus einem Signal-/Rauschverhältnis, einem Signaloffset oder einem Signalmorphologiekriterium, wie einer Signalanstiegsgeschwindigkeit oder der Anzahl der Nullliniendurchgänge innerhalb eines Zeitfensters, ergeben.

Ergibt die Signalqualitätsüberprüfung, dass die zur Ermittlung des Erregungszustands vorgesehenen Signale keine ausreichende Qualität aufweisen, kann die zur Stimulationserfolgskontrolle eingesetzte Klassifikation und nachfolgende automatische Anpassung mindestens eines Steuerungsparameters der Anordnung zumindest teilweise temporär abgeschaltet werden. Alternativ ist es auch möglich, das erhaltene Klassifikationsergebnis entsprechend zu kennzeichnen, dort also anzugeben, dass dieses Ergebnis auf der Grundlage von Signalen getroffen wurde, die die durchgeführte Signalqualitätsüberprüfung nicht bestanden haben. Damit wird die entsprechende Klassifikation als weniger verlässlich im Vergleich zu anderen Klassifikationen gekennzeichnet.

In einer Variante veranlasst das Programm den Prozessor dazu, in Abhängigkeit der durchgeführten Signalqualitätsüberprüfung automatisch einen Signalverarbeitungsparameter der Anordnung anzupassen. Dabei kann sich beispielsweise um eine Verstärkung, eine Dämpfung, eine Filterung, eine Differenzierung, eine Glättung, eine Mittelung, eine Phasenverschiebung oder eine Offsetkorrektur der gemessenen Signale handeln, um eine bessere Auswertung dieser Signale erreichen zu können. Auf diese Weise wird es möglich, Signale, die die Signalqualitätsüberprüfung möglicherweise nicht oder nur knapp bestehen, derart zu verstärken bzw. vom Untergrund abzuheben, dass doch noch eine ausreichende Qualität vorliegt, die Signalqualitätsüberprüfung also bestanden wird, sodass eine verlässliche Auswertung der entsprechenden Signale ermöglicht wird.

In einer Variante veranlasst das Programm den Prozessor dazu, ein morphologisches Analysekriterium aus einem abgeleiteten Elektrokardiogramm oder ein morphologisches Analysekriterium aus einem Elektrokardiogramm, das mittels einer der Stimulationseinheit zugehörigen Elektrode erhalten wurde, zur Klassifikation des Erregungszustands heranzuziehen. Auf diese Weise kann beispielsweise eine Mustererkennung in dem gewonnenen Elektrokardiogramm durchgeführt werden, um bestimmte Muster zu identifizieren und daraus entsprechende Aussagen hinsichtlich eines Stimulationserfolgs vornehmen zu können.

In einer Variante veranlasst das Programm den Prozessor dazu, eine Breite des QRS-Komplexes im Elektrokardiogramm oder ein zu dieser QRS-Komplexbreite korreliertes Analysekriterium zur Klassifikation des Erregungszustands heranzuziehen. Denn aus der Breite des QRS-Komplexes lässt sich ablesen, ob eine selektive His-Bündel-Stimulation oder eine nicht-selektive His-Bündel-Stimulation erfolgt ist.

In einer Variante veranlasst das Programm den Prozessor dazu, einen zeitlichen Versatz zwischen der Ausführung der kardialen Stimulation auf der einen Seite und der Detektion des kardialen elektrischen Signals auf der anderen Seite zur Klassifikation des Erregungszustands heranzuziehen. Mit anderen Worten ausgedrückt, wird in dieser Variante die Zeit, die zwischen der Stimulation und der detektierten kardialen Erregung verstreicht, berücksichtigt, um eine entsprechende Klassifikation des Erregungszustands vornehmen zu können. Denn die unterschiedlichen kardialen Erregungszustände bereiten sich unterschiedlich schnell aus. Daher ist der Zeitpunkt, zu dem eine kardiale Erregung nach einer vorherigen Stimulation detektiert werden kann, mitunter ein wichtiges Kriterium, um die detektierte Erregung einer bestimmten Klasse zuordnen zu können.

In einer Variante veranlasst das Programm den Prozessor dazu, zur Klassifikation des Erregungszustands zumindest einen Signalverlauf des Elektrokardiogramms heranzuziehen, wobei das Elektrokardiogramm in dieser Variante ein unipolar abgeleitetes Elektrokardiogramm von einem Pol der Elektrode der Stimulationseinheit gegen eine großflächige Körperelektrode (beispielsweise ein Gehäuse der Anordnung zur Stimulation des Herzens) des betreffenden Patienten ist.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn er auf dem Prozessor ausgeführt wird.

Zunächst wird eine kardiale Stimulation mittels einer Stimulationseinheit ausgeführt.

Anschließend wird mittels einer Detektionseinheit ein kardiales elektrisches Signal detektiert. Dieses Signal wurde durch eine kardiale Erregung infolge der zuvor durchgeführten kardialen Stimulation erzeugt.

Anschließend wird der Erregungszustand des durch die kardiale Stimulation stimulierten Herzens anhand des detektierten elektrischen Signals ermittelt.

Nun wird der Erregungszustand in eine von mindestens drei Klassen klassifiziert. Dabei umfassen die mindestens drei Klassen eine erste Klasse, die repräsentativ für einen ersten Erregungszustand ist, eine zweite Klasse, die repräsentativ für einen zweiten Erregungszustand ist, der unterschiedlich zu dem ersten Erregungszustand ist, und eine dritte Klasse, die repräsentativ für eine erfolglose Stimulation ohne detektierbaren Erregungszustand repräsentativ ist.

Nachfolgend erfolgt eine automatische Anpassung mindestens eines Steuerparameters einer implantierbaren Anordnung zur Stimulation eines menschlichen oder tierischen Herzens. Diese automatische Anpassung erfolgt dabei in Abhängigkeit der zuvor durchgeführten Klassifikation. Bei dem Steuerparameter handelt es sich um eine His-Bündel-Stimulationsenergie, einen His-Bündel-Stimulationsvektor, eine Betriebsart der zur Stimulation eingesetzten Anordnung, mindestens einen Parameter eines Zeitgebers der Anordnung oder einen Stimulationssteuerparameter, der für eine kardiale Resynchronisationstherapie geeignet ist.

Ein weiterer Aspekt der vorliegenden Erfindung betrifft ein medizinisches Verfahren zur Behandlung eines menschlichen oder tierischen Patienten, der eine solche Behandlung benötigt. Dieses Verfahren wird mittels einer implantierbaren Anordnung zur Stimulation des Herzen des Patienten durchgeführt. Die Anordnung weist einen Prozessor, eine Speichereinheit, eine Stimulationseinheit zum Stimulieren des His-Bündels des Herzens des Patienten und eine Detektionseinheit zum Detektieren eines elektrischen Signals des Herzens des Patienten auf. Das Verfahren umfasst dabei die nachfolgend erläuterten Schritte.

Zunächst wird eine kardiale Stimulation des Herzens des Patienten mittels der Stimulationseinheit durchgeführt.

Nachfolgend wird ein kardiales elektrisches Signal mittels der Detektionseinheit detektiert. Dabei wurde das kardiale elektrische Signal durch die zuvor durchgeführte kardiale Stimulation ausgelöst bzw. erzeugt.

Dann erfolgt die Ermittlung eines Erregungszustands des durch die kardiale Stimulation studierten Herzens des Patienten. Diese Ermittlung erfolgt auf der Grundlage des zuvor detektierten elektrischen Signals.

Nun wird der ermittelte Erregungszustand in eine von mindestens drei Klassen klassifiziert. Diese mindestens drei Klassen umfassen eine erste Klasse für einen ersten Erregungszustand, eine zweite Klasse für einen zweiten Erregungszustand und eine dritte Klasse für eine erfolglose Stimulation ohne detektierbaren Erregungszustand. Der zweite Erregungszustand unterscheidet sich dabei von dem ersten Erregungszustand.

Nach der Klassifikation erfolgt eine automatische Anpassung mindestens eines Steuerparameters der Anordnung in Abhängigkeit der durchgeführten Klassifikation. Der Steuerparameter kann dabei eine His-Bündel-Stimulationsenergie, ein His-Bündel-Stimulationsvektor, eine Betriebsart der implantierbaren Anordnung, mindestens ein Parameter eines Zeitgebers der Anordnung oder ein Stimulationssteuerparameter, der für eine kardiale Resynchronisationstherapie geeignet ist, sein.

Ein Aspekt der vorliegenden Erfindung betrifft eine Analyseeinrichtung zur Unterstützung der Implantation einer Anordnung zur Stimulation des menschlichen oder tierischen Herzens mit den nachfolgend erläuterten Merkmalen. Eine derartige Analyseeinrichtung weist einen Prozessor und eine Speichereinheit auf.

Erfindungsgemäß weist die Speichereinheit ein computerlesbares Programm auf, das den Prozessor dazu veranlasst, die nachfolgend erläuterten Schritte auszuführen, wenn es auf dem Prozessor ausgeführt wird.

Zunächst wird ein Elektrokardiogramm eines menschlichen oder tierischen Herzens, in das eine Anordnung zur Stimulation dieses Herzens implantiert wird, empfangen. Bei dem Elektrokardiogramm kann es sich um ein Elektrokardiogramm beliebigen Typs handeln. Besonders geeignete Elektrokardiogramme sind intrakardiale Elektrogramme.

Anschließend erfolgt eine automatische Identifikation von Signalen des Elektrokardiogramms, die durch eine His-Bündel-Stimulation verursacht werden. Diese Signale erscheinen jeweils zwischen einem atrialen Signal und einem ventrikulären Signal.

Gemäß vorteilhaften Ausführungsformen der vorliegenden Erfindung können Signale des Elektrokardiogramms, die durch eine HIS-Bündel Stimulation ausgelöst werden, durch folgende Schritte identifiziert werden:
a) Vorverarbeitung des EKG-Signals durch Verstärkung, eine Dämpfung, eine Filterung, eine Differenzierung, eine Glättung, eine Mittelung, eine Phasenverschiebung und/oder eine Offsetkorrektur,
b) Auswertung der EKG-Signalmorphologie zur Erkennung HIS-Bündel-typischer Signale. Das HIS-Signal erscheint nach einem atrialen Signal und vor einem ventrikulären Signal; daher kann gemäß Ausführungsformen der Erfindung das HIS-Signal zwischen diesen identifiziert werden. Gemäß weiteren Aspekten der Erfindung kann eine Unterscheidung des atrialen und ventrikulären Signals mittels Auswertung der Signalamplituden und Verhältnisse zueinander erfolgen. Bei geeigneter HIS-Position ist die Amplitude des atrialen Signals kleiner als die Amplitude des HIS-Signals, und die Amplitude des ventrikulären Signals deutlich größer (z.B. 2-10fach) als die Amplitude des HIS-Signals.

Nachfolgend werden die zuvor identifizierten Signale in dem empfangenen Elektrokardiogramm markiert.

Dann wird das derart markierte Elektrokardiogramm auf einem Ausgabegerät ausgegeben. Bei dem Ausgabegerät kann es sich um ein Bauelement der Analyseeinrichtung oder um ein separates Ausgabegerät, mit dem die Analyseeinrichtung verbunden ist, handeln.

Die vorgenommene Markierung kann beispielsweise optisch oder akustisch erfolgen. Eine optische Markierung lässt sich beispielsweise besonders einfach dadurch realisieren, dass die identifizierten His-Bündel-spezifischen Signale farblich hervorgehoben werden. Auf diese Weise wird es einem Anwender der Analyseeinrichtung signifikant erleichtert, His-Bündel-spezifische Signale innerhalb des Elektrokardiogramms zu erkennen und anhand der Güte bzw. Ausprägung dieser His-Bündel-spezifischen Signale eine korrekte Positionierung einer zur His-Bündel-Stimulation vorgesehenen Elektrode innerhalb eines menschlichen oder tierischen Herzens vorzunehmen.

Durch den Einsatz einer derartigen Analyseeinrichtung ist es nicht mehr erforderlich, während der Implantation eines Herzschrittmachers oder einer anderen Anordnung zur Stimulation des menschlichen oder tierischen Herzens eine elektrophysiologische Untersuchung unter Verwendung eines Elektrophysiologie-Messeplatzes durchzuführen. Vielmehr ist eine derartige gesonderte Untersuchung nicht erforderlich. Stattdessen kann durch den Einsatz des erfindungsgemäß beanspruchten Analysegeräts unmittelbar während der Implantation überprüft werden, wie gut, das heißt in welchem Ausmaß, das His-Bündel des betreffenden Herzens durch die entsprechende Elektrode kontaktiert wird und ob eine ausreichende Stimulation des His-Bündels bei der gewählten Positionierung der Elektrode gewährleistet ist.

Das Ausgabegerät kann beispielsweise ein Monitor oder eine Kombination aus einem Monitor und einem Lautsprecher sein. Das Ausgabegerät kann alternativ auch ein Drucker sein, der zum Ausdrucken eines markierten Elektrokardiogramms auf Papier oder einem anderen Medium geeignet ist.

Die Analyseeinrichtung kann auch als Pace-Sense-Analysator (PSA) bezeichnet werden.

Die Analyseeinrichtung kann in einer Variante in Form eines separaten Gerätes ausgeführt sein. In einer anderen Variante ist die Analyseeinrichtung Teil einer Anordnung zur Stimulation des menschlichen oder tierischen Herzens. Sie kann in einem solchen Fall auch als Analysemodul bezeichnet werden. Dabei ist es in dieser Variante vorgesehen, dass die Analyseeinrichtung keinen separaten Prozessor und keine separate Speichereinheit aufweist, sondern auf einen Prozessor und eine Speichereinheit der Anordnung zur Stimulation des menschlichen oder tierischen Herzens zurückgreift. Somit lässt sich die zusätzliche Möglichkeit einer Analyse einer korrekten Elektrodenpositionierung in dieser Variante ohne zusätzlichen apparativen Aufwand realisieren. Dadurch wird die Implantation von Herzschrittmachern, die zur His-Bündel-Stimulation geeignet sind, erheblich vereinfacht.

In einer Variante weist die Analyseeinrichtung eine Stimulationseinheit zum Stimulieren des His-Bündels eines menschlichen oder tierischen Herzens sowie eine Detektionseinheit zum Detektieren eines elektrischen Signals desselben Herzens auf. In dieser Variante veranlasst das Programm den Prozessor dazu, die nachfolgend erläuterten Schritte auszuführen, wenn es auf dem Prozessor ausgeführt wird.

Zunächst wird eine kardiale Stimulation mittels der Stimulationseinheit ausgeführt. Dabei ist diese kardiale Stimulation zur Ermittlung der Reizschwelle des His-Bündels geeignet.

Nachfolgend wird ein kardiales elektrisches Signal detektiert, das durch die vorherige kardiale Erregung infolge der durchgeführten kardialen Stimulation erzeugt wurde. Hierzu wird die Detektionseinheit verwendet.

Anschließend wird ein Erregungszustand des durch die kardiale Stimulation stimulierten Herzens anhand des detektierten elektrischen Signals ermittelt.

Dieser Erregungszustand wird dann in eine von mindestens drei Klassen klassifiziert. Dabei umfassen diese mindestens drei Klassen eine erste Klasse, die einen ersten Erregungszustand des Herzens angibt. Sie umfassen ferner eine zweite Klasse, die einen zweiten Erregungszustand des Herzens angibt, der sich von dem ersten Erregungszustand unterscheidet. Schließlich umfassen sie eine dritte Klasse, die eine erfolglose Stimulation ohne detektierbaren Erregungszustand angibt.

Durch eine derartige Klassifizierung eines detektierten Erregungszustands kann besonders leicht erkannt werden, ob eine ausreichende Kontaktierung des His-Bündels mittels einer Elektrode eines Herzschrittmachers bzw. einer anderen Anordnung zur Stimulation eines Herzens erreicht wurde.

Geeignete Stimulationsamplituden der von der Stimulationseinheit zur Ermittlung der Reizschwelle des His-Bündels abgegebenen Impulse liegen in einer Variante in einem Bereich von 12 V bis 30 V, insbesondere 15 V bis 25 V, insbesondere 20 V bis 22 V.

Geeignete Pulsbreiten der von der Stimulationseinheit zur Ermittlung der Reizschwelle des His-Bündels abgegebenen Impulse liegen in einem Bereich zwischen 1 ms und 15 ms, insbesondere zwischen 2 ms und 12 ms, insbesondere zwischen 3 ms und 10 ms, insbesondere zwischen 4 ms und 9 ms, insbesondere zwischen 5 ms und 8 ms.

Wie bereits erwähnt, handelt es sich bei einer der mindestens drei Klassen um eine Klasse, die für eine erfolglose Stimulation ohne detektierbaren Erregungszustand vorgesehen ist. Die übrigen Klassen der mindestens drei Klassen (also die erste Klasse, die zweite Klasse sowie eine optional vorgesehene weitere Klasse) sind in einer Variante aus den nachfolgend erläuterten Klassen ausgewählt. Eine Klasse betrifft einen Erregungszustand des Herzens, für den ausschließlich eine Stimulation des His-Bündels des stimulierten Herzens erfolgt ist. Das heißt, in diese Klasse fällt ein Erregungszustand, bei dem eine selektive Stimulation des His-Bündels erfolgt ist. Eine weitere Klasse betrifft einen Erregungszustand, für den sowohl eine Stimulation des His-Bündels des Herzens als auch eine Stimulation des ventrikulären Myokards erfolgt ist. Somit betrifft diese Klasse einen Erregungszustand, der auf einer nicht-selektiven Stimulation beruht. Eine Klasse betrifft einen Erregungszustand, für den ausschließlich eine Stimulation des ventrikulären Myokards des stimulierten Herzens erfolgt ist. Das heißt, dieser Erregungszustand beruht auf einer Stimulation, bei der das His-Bündel nicht erfasst wurde. Er kann als rein ventrikulärer Erregungszustand bezeichnet werden. Eine Klasse betrifft einen Erregungszustand, bei dem eine His-Bündel-Stimulation und eine Stimulation linksventrikulärer Leitungsbahnen erfolgt sind. Eine weitere Klasse betrifft einen Erregungszustand, bei dem eine His-Bündel-Stimulation ohne Stimulation linksventrikulärer Leitungsbahnen erfolgt ist. Eine derartige Stimulation wird auch als Rechtsschenkelblock bzw. Rechtsschenkelblockbild (RSB bzw. RBBB) bezeichnet.

Die Klassifikation des Erregungszustandes in eine der vordefinierten Klassen bedeutet automatisch eine Klassifikation der dem Erregungszustand zu Grunde liegenden Stimulation in eine derartige Klasse.

In einer Variante veranlasst das Programm den Prozessor dazu, einen automatischen Reizschwellentest (ATM) durchzuführen. Im Rahmen eines derartigen Reizschwellentest wird eine Reizschwelle für eine erfolgreiche His-Bündel-Stimulation bestimmt. Dabei wird eine selektive oder nicht selektive His-Bündel-Stimulation als erfolgreich angesehen, während eine Stimulation, bei der das His-Bündel nicht erfasst ist bzw. die lediglich eine ventrikuläre Stimulation zur Folge hat, als nicht erfolgreich angesehen werden.

In einer Variante veranlasst das Programm den Prozessor dazu, jede mittels der Stimulationseinheit ausgeführte kardiale Stimulation zu klassifizieren. Hierfür kann beispielsweise eine aktive Erfassungskontrolle (ACC), wahlweise unter Anwendung eines Beat-to-Beat-Algorithmus, angewendet werden.

In einer Variante veranlasst das Programm den Prozessor dazu, eine Breite des QRS-Komplexes im Elektrokardiogramm oder ein zu dieser QRS-Komplexbreite korreliertes Analysekriterium zur Klassifikation des Erregungszustands heranzuziehen. Denn aus der Breite des QRS-Komplexes lässt sich ablesen, ob eine selektive His-Bündel-Stimulation oder eine nicht-selektive His-Bündel-Stimulation erfolgt ist.

In einer Variante veranlasst das Programm den Prozessor dazu, einen zeitlichen Versatz zwischen der Ausführung der kardialen Stimulation auf der einen Seite und der Detektion des kardialen elektrischen Signals auf der anderen Seite zur Klassifikation des Erregungszustands heranzuziehen. Mit anderen Worten ausgedrückt, wird in dieser Variante die Zeit, die zwischen der Stimulation und der detektierten kardialen Erregung verstreicht, berücksichtigt, um eine entsprechende Klassifikation des Erregungszustands vornehmen zu können. Denn die unterschiedlichen kardialen Erregungszustände bereiten sich unterschiedlich schnell aus. Daher ist der Zeitpunkt, zu dem eine kardiale Erregung nach einer vorherigen Stimulation detektiert werden kann, mitunter ein wichtiges Kriterium, um die detektierte Erregung einer bestimmten Klasse zuordnen zu können.

In einer Variante veranlasst das Programm den Prozessor dazu, zur Klassifikation des Erregungszustands zumindest einen Signalverlauf des Elektrokardiogramms heranzuziehen, wobei das Elektrokardiogramm in dieser Variante ein unipolar abgeleitetes Elektrokardiogramm von einem Pol der Elektrode der Stimulationseinheit gegen eine großflächige Körperelektrode des betreffenden Patienten ist.

In einer Variante ist die Analyseeinrichtung mit einer Detektionseinheit ausgestattet. Diese Detektionseinheit kann besonders geeignet sein, His-Bündel-spezifische Erregungsimpulse des Herzens zu detektieren oder aber spezifisch dafür ausgestaltet und hergerichtet sein.

Zu diesem Zweck weist die Detektionseinheit in einer Variante einen Tiefpassfilter auf, der für Signale, die durch eine Erregung des His-Bündels erzeugt werden, besonders geeignet ist. Ein solcher Tiefpassfilter weist eine Grenzfrequenz von mindestens 100 kHz (also 100 kHz oder größer), insbesondere mindestens 10 kHz, insbesondere mindestens 1 kHz, insbesondere mindestens 500 Hz, insbesondere mindestens 200 Hz und ganz besonders mindestens 100 Hz auf. In einer Variante weist ein solcher Tiefpassfilter eine Grenzfrequenz auf, die in einem Bereich zwischen 100 Hz und 100 kHz, insbesondere zwischen 200 Hz und 10 kHz, insbesondere zwischen 500 Hz und 1 kHz liegt. Signalanteile mit einer Frequenz unterhalb der Grenzfrequenz lässt ein solcher Tiefpassfilter im Wesentlichen ungehindert passieren. Demgegenüber werden Signalanteile mit einer Frequenz, die größer als die Grenzfrequenz ist, abgeschwächt bzw. gedämpft.

Die besondere Eignung der Detektionseinheit zur Detektion von His-Bündel-spezifischen Pulsen bzw. die Ausbildung und Herrichtung der Detektionseinheit für diesen Zweck kann auch durch eine Abtastrate realisiert sein, die in einer Variante mindestens 500 Hz, insbesondere mindestens 1 kHz, insbesondere mindestens 2 kHz, insbesondere mindestens 5 kHz, insbesondere mindestens 10 kHz, insbesondere mindestens 100 kHz, insbesondere mindestens 1 MHz beträgt. In einer Variante liegt die Abtastrate der Detektionseinheit in einem Frequenzbereich zwischen 500 Hz und 1 MHz, insbesondere zwischen 1 kHz und 100 kHz, insbesondere zwischen 2 kHz und 10 kHz, insbesondere zwischen 3 kHz und 5 kHz.

In einer Variante weist die Detektionseinheit eine Empfindlichkeit auf, die in einem Bereich zwischen 0,01 mV und 0,25 mV, insbesondere zwischen 0,05 mV und 0,2 mV, insbesondere zwischen 0,1 mV und 0,15 mV liegt. Eine derartige Empfindlichkeit der Detektionseinheit ist besonders gut geeignet, um His-Bündel-spezifische elektrische kardiale Signale detektieren zu können.

In einer Variante veranlasst das Programm den Prozessor dazu, ein morphologisches Analysekriterium aus dem Elektrokardiogramm für die automatische Identifikation der Signale, die durch eine His-Bündel-Stimulation verursacht werden, heranzuziehen. Auf diese Weise kann beispielsweise eine Mustererkennung in dem gewonnenen Elektrokardiogramm durchgeführt werden, um bestimmte Muster zu identifizieren und somit die Identifikation His-Bündel-spezifischer Signale zu ermöglichen.

In einer Variante weist die Analyseeinrichtung eine erste Stimulationseinheit zum Stimulieren des His-Bündels eines menschlichen oder tierischen Herzens, eine zweite Stimulationseinheit zum Stimulieren einer vom His-Bündel verschiedenen Herzregionen desselben Herzens, eine erste Detektionseinheit und eine zweite Detektionseinheit auf, die jeweils zum Detektieren eines elektrischen Signals desselben Herzens geeignet sind. Dabei ist eine Elektrode der zweiten Stimulationseinheit insbesondere dafür vorgesehen, an einem atrialen oder ventrikulären Implantationsort implantiert zu werden, um als konventionelle Herzelektrode eine atriale oder ventrikuläre Stimulation (also keine Stimulation des His-Bündels) vorzunehmen. Die erste Detektionseinheit ist insbesondere dafür vorgesehen, eine Stimulation des His-Bündels des Herzens zu erfassen. Die zweite Detektionseinheit ist demgegenüber dafür vorgesehen, eine Stimulation einer Herzregion durch die zweite Stimulationseinheit zu erfassen. Wenn die zweite Stimulationseinheit zur Stimulation eines Atriums des Herzens vorgesehen ist, handelt es sich somit bei der zweiten Detektionseinheit insbesondere um eine atriale Detektionseinheit. Wenn die zweite Stimulationseinheit zur Stimulation eines ventrikulären Herzbereiches vorgesehen ist, handelt es sich bei der zweiten Detektionseinheit insbesondere um eine ventrikuläre Detektionseinheit. In dieser Variante wird es also möglich, nicht nur das His-Bündel eines Herzens zu stimulieren und die entsprechende Stimulation zu detektieren, sondern auch eine atriale und/oder ventrikuläre Stimulation mit anschließender Detektion entsprechender kardialer Signale durchzuführen.

In einer Variante veranlasst das Programm den Prozessor dazu, bei einer Detektion einer intrinsischen Erregung eines der beiden Atrien des stimulierten Herzens durch die erste Detektionseinheit oder die zweite Detektionseinheit eine Stimulation des His-Bündels mittels der ersten Stimulationseinheit auszulösen. Es ist durch das Vorsehen mehrerer Stimulationseinheiten und mehrerer Detektionseinheiten also möglich, eine Stimulation des His-Bündels in Abhängigkeit anderer kardialer Stimulationen zu triggern. Dadurch lässt sich eine besonders effektive Stimulation des His-Bündels erreichen, wodurch ein positiver Einfluss auf die durch eine entsprechende His-Bündel-Stimulation erzeugten kardialen Signale und deren anschließende Detektion erzielt werden kann.

In einer Variante ist die Analyseeinrichtung als Einkanalstimulator ausgestaltet oder Teil eines solchen Einkanalstimulators. Ein derartiger Einkanalstimulator weist nur einen einzigen Anschluss für eine Stimulations- und Detektionselektrode auf. Dabei ist die Stimulations- und Detektionselektrode Teil der Stimulationseinheit und der Detektionseinheit. Somit kann sie auch als His-Bündel-Stimulations- und Detektionselektrode oder als His-Bündel-Stimulations- und Detektionskanal bezeichnet werden. Dabei ist die Detektionseinheit nicht nur zur Detektion von His-Bündel-spezifischen kardialen Signalen des zu stimulierenden Herzens vorgesehen und eingerichtet. Vielmehr ist sie auch dafür vorgesehen und eingerichtet, eine intrinsische Erregung eines der beiden Atrien des zu stimulierenden Herzens zu detektieren. Signale, die aus einer derartigen intrinsischen Erregung eines der beiden Atrien resultieren, werden auch als P-Welle bezeichnet. Wenn die Detektionseinheit zur Detektion derartiger atrialer Signale vorgesehen und eingerichtet ist, lässt sich die Stimulation des His-Bündels des zu stimulierenden Herzens durch die Stimulationseinheit besonders leicht in Abhängigkeit dieser intrinsischen atrialen Signale triggern bzw. ausführen.

In einer Variante ist die Analyseeinrichtung als Zweikanalstimulator ausgestaltet oder Teil eines solchen Zweikanalstimulators. Sie weist dann einen ersten Anschluss für eine His-Bündel-Stimulations- und Detektionselektrode (bzw. für einen His-Bündel-Stimulations- und Detektionskanal) und einen zweiten Anschluss auf, der als atrialer Stimulations- und Detektionskanal oder als ventrikulärer Stimulations- und Detektionskanal zum Anschluss einer entsprechenden atrialen Stimulations- und Detektionselektrode oder einer ventrikulären Stimulations- und Detektionselektrode ausgestaltet sein kann. In solch einer Variante weist die Anordnung neben der Stimulationseinheit und der Detektionseinheit typischerweise eine zweite Stimulationseinheit und eine zweite Detektionseinheit auf, der die atriale oder ventrikuläre Stimulations- und Detektionselektrode dann zugeordnet ist.

In einer Variante ist die Analyseeinrichtung als Dreikanalstimulator ausgestaltet oder ein Teil eines derartigen Dreikanalstimulators. Ein derartiger Dreikanalstimulator weist einen ersten Anschluss für einen His-Bündel-Stimulations- und Detektionskanal (bzw. für eine His-Bündel-Stimulations- und Detektionselektrode), einen zweiten Anschluss für einen atrialen Stimulations- und Detektionskanal (bzw. für eine atriale Stimulations- und Detektionselektrode) und einen dritten Anschluss für einen ventrikulären Stimulations- und Detektionskanal (bzw. für eine ventrikuläre Stimulations- und Detektionselektrode auf). Mit einem derartigen Dreikanalstimulator können also sowohl His-Bündel-spezifische Stimulationen durchgeführt und His-Bündel-spezifische Signale erfasst werden als auch atriale und ventrikuläre Stimulationen durchgeführt und atriale und ventrikuläre Signale besonders leicht detektiert werden.

In einer Variante ist die Analyseeinrichtung als Vierkanalstimulator ausgestaltet oder Teil eines derartigen Vierkanalstimulators. Ein derartiger Vierkanalstimulator weist einen ersten Anschluss für einen His-Bündel-Stimulations- und Detektionskanal (bzw. für eine His-Bündel-Stimulations- und Detektionselektrode), einen zweiten Anschluss für einen atrialen Stimulations- und Detektionskanal (bzw. für eine atriale Stimulations- und Detektionselektrode), einen dritten Anschluss für einen ersten ventrikulären Stimulations- und Detektionskanal (bzw. eine erste ventrikuläre Stimulations- und Detektionselektrode) und einen vierten Anschluss für einen zweiten ventrikulären Stimulations- und Detektionskanal (bzw. für eine zweite ventrikuläre Stimulations- und Detektionselektrode) auf. Mit einem derartigen Vierkanalstimulator können also beispielsweise beide Ventrikel eines menschlichen oder tierischen Herzens gleichzeitig stimuliert werden. In gleicher Weise können gleichzeitig Signale aus beiden Ventrikeln erfasst werden. Darüber hinaus ist die bereits in Zusammenhang mit den vorherigen Varianten erläuterte His-Bündel-spezifische Stimulation und Detektion sowie eine atriale Stimulation und Detektion möglich.

In einer weiteren Variante ist die Analyseeinrichtung als Fünfkanalstimulator ausgestaltet oder Teil eines derartigen Fünfkanalstimulators. Ein solcher Fünfkanalstimulator weist insgesamt fünf Anschlüsse auf. Ein erster Anschluss ist für einen His-Bündel-Stimulations- und Detektionskanal (bzw. für eine His-Bündel-Stimulations- und Detektionselektrode) vorgesehen. Ein zweiter Anschluss ist für einen atrialen Stimulations- und Detektionskanal (bzw. für eine atriale Stimulations- und Detektionselektrode) vorgesehen. Ein dritter Anschluss dient zum Anschließen eines ersten ventrikulären Stimulations- und Detektionskanals (bzw. einer ersten ventrikulären Stimulations- und Detektionselektrode). Ein vierter Anschluss ist für einen zweiten ventrikulären Stimulations- und Detektionskanal (bzw. für eine zweite ventrikuläre Stimulations- und Detektionselektrode) vorgesehen. Schließlich ist ein fünfter Anschluss für einen dritten ventrikulären Stimulations- und Detektionskanal (bzw. für eine dritte ventrikuläre Stimulations- und Detektionselektrode) vorgesehen. So kann die implantierbare Anordnung beispielsweise als Stimulator eingesetzt werden, der für die kardiale Resynchronisation (CRT) eingerichtet ist (CRT-Stimulator) und der einen quadropolaren linksventrikulären Kanal aufweist. Alternativ kann die implantierbare Anordnung eine multipolare Stimulation (ein sogenanntes Multipol-Pacing) unter Verwendung eines His-Bündel-spezifischen Stimulationskanals ermöglichen.

In einer Variante weist die Analyseeinrichtung eine Einrichtung zur automatischen Kardioversion und/oder Defibrillation des Herzens auf oder ist ein Teil einer derartigen Einrichtung. Sie ist dann mit entsprechenden Anschlüssen ausgestattet, um diese Einrichtung mit geeigneten Herzregionen zu verbinden.

In einer Variante veranlasst das Programm den Prozessor dazu, vor der Ermittlung des Erregungszustands eine Signalqualitätsüberprüfung (nach dem englischen Fachbegriff "signal quality check" auch als SQC abgekürzt) durchzuführen. Mit einer derartigen Signalqualitätsüberprüfung lässt sich feststellen, ob die zur Ermittlung des Erregungszustands vorgesehenen Signale zur Auswertung geeignet sind. Diese Eignung kann sich beispielsweise aus der absoluten Signalintensität oder aus einem Signal-/Rauschverhältnis ergeben.

Ergibt die Signalqualitätsüberprüfung, dass die zur Ermittlung des Erregungszustands vorgesehenen Signale keine ausreichende Qualität aufweisen, kann die vorgesehene Klassifikation zumindest teilweise temporär abgeschaltet werden. Alternativ ist es auch möglich, das erhaltene Klassifikationsergebnis entsprechend zu kennzeichnen, dort also anzugeben, dass dieses Ergebnis auf der Grundlage von Signalen getroffen wurde, die die durchgeführte Signalqualitätsüberprüfung nicht bestanden haben. Damit wird die entsprechende Klassifikation als weniger verlässlich im Vergleich zu anderen Klassifikationen gekennzeichnet.

In einer Variante veranlasst das Programm den Prozessor dazu, in Abhängigkeit der durchgeführten Signalqualitätsüberprüfung automatisch einen Signalverarbeitungsparameter der Anordnung anzupassen. Dabei kann sich beispielsweise um eine Verstärkung, eine Dämpfung, eine Filterung, eine Differenzierung, eine Glättung, eine Mittelung, eine Phasenverschiebung oder eine Offsetkorrektur der gemessenen Signale handeln, um eine bessere Auswertung dieser Signale erreichen zu können. Auf diese Weise wird es möglich, Signale, die die Signalqualitätsüberprüfung möglicherweise nicht oder nur knapp bestehen, derart zu verstärken bzw. vom Untergrund abzuheben, dass doch noch eine ausreichende Qualität vorliegt, die Signalqualitätsüberprüfung also bestanden wird, sodass eine verlässliche Auswertung der entsprechenden Signale ermöglicht wird.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn er auf dem Prozessor ausgeführt wird.

Zunächst wird ein Elektrokardiogramm eines menschlichen oder tierischen Herzens, in das eine Anordnung zur Stimulation dieses Herzens implantiert wird, empfangen.

Anschließend erfolgt eine automatische Identifikation von Signalen des Elektrokardiogramms, die durch eine His-Bündel-Stimulation verursacht werden. Diese Signale erscheinen jeweils zwischen einem atrialen Signal und einem ventrikulären Signal.

Nachfolgend werden die zuvor identifizierten Signale in dem empfangenen Elektrokardiogramm markiert.

Im Anschluss daran wird das derart markierte Elektrokardiogramm auf einem geeigneten Ausgabegerät ausgegeben.

Ein derartiges Computerprogrammprodukt dient zur softwaretechnischen Realisierung derjenigen Verfahrensschritte, die auch von der erfindungsgemäß beanspruchten Analyseeinrichtung durchgeführt werden. Mithin lässt sich eine derartige Analyse kardialer Signale zur Unterstützung der Implantation einer Anordnung zur Stimulation des menschlichen oder tierischen Herzens entweder hardwaretechnisch (in Form der erfindungsgemäß beanspruchten Analyseeinrichtung) oder softwaretechnisch (in Form des erfindungsgemäß beanspruchten Computerprogrammprodukts) realisieren.

Ein Aspekt der vorliegenden Erfindung betrifft ein Verfahren zur Auswertung von Daten, die bei einer Implantation einer Anordnung zur Stimulation des menschlichen oder tierischen Herzens erhalten werden. Bei diesem Verfahren handelt es sich um ein reines Datenauswerteverfahren, das ohne nähere Interaktion mit einem menschlichen oder tierischen Körper durchgeführt werden kann.

Für dieses Datenauswerteverfahren wird ein Elektrokardiogramm eines menschlichen oder tierischen Herzens, in das eine Anordnung zur Stimulation dieses Herzens implantiert wird, empfangen.

Nachfolgend werden Signale des Elektrokardiogramms, die durch eine His-Bündel-Stimulation verursacht werden, automatisch identifiziert.

Die derart identifizierten Signale werden in dem empfangenen Elektrokardiogramm markiert.

Das derart markierte Elektrokardiogramm wird dann auf einem geeigneten Ausgabegerät ausgegeben.

Ein Aspekt der vorliegenden Erfindung betrifft ein Verfahren zur Implantation einer Anordnung zur Stimulation des menschlichen oder tierischen Herzens. Diese Implantation wird bei einem Patienten vorgenommen, der eine solche Anordnung benötigt. Das Implantationsverfahren umfasst die nachfolgend erläuterten Schritte.

Zunächst wird ein Bereich des Herzens des Patienten, in dem das His-Bündel des Herzens vermutet wird, mit einer Elektrode kontaktiert. Diese Elektrode ist Teil einer Stimulationseinheit zum Stimulieren des His-Bündels eines menschlichen oder tierischen Herzens.

Nachfolgend wird eine Stimulation des His-Bündels mittels der Stimulationseinheit durchgeführt.

Daraufhin wird ein Elektrokardiogramm mittels einer Detektionseinheit detektiert. Wenn die vorherige His-Bündel-Stimulation mittels der Stimulationseinheit erfolgreich war, finden sich in diesem Elektrokardiogramm His-Bündel-spezifische Signale.

Diese His-Bündel-spezifischen Signale werden nun in dem Elektrokardiogramm automatisch identifiziert.

Die zuvor identifizierten Signale werden in dem empfangenen Elektrokardiogramm markiert. Auf diese Weise wird ein markiertes Elektrokardiogramm erhalten.

Das markierte Elektrokardiogramm wird auf einem Ausgabegerät ausgegeben.

Nun kann die Position der Elektrode in Abhängigkeit der markierten Signale angepasst werden. Eine derartige Anpassung ist nicht erforderlich, wenn die His-Bündel-spezifischen Signale, die im Elektrokardiogramm markiert sind, bereits einem vordefinierten bzw. vorgebbaren Gütekriterium entsprechen. Wenn die markierten Signale beispielsweise ausreichend groß sind, deutet dies auf eine gute Kontaktierung des His-Bündels mittels Elektrode hin. Eine Anpassung der Position der Elektrode erübrigt sich dann.

Wenn hingegen die Signale noch keine ausreichende Kontaktierung des His-Bündels anzeigen (weil sie entweder insgesamt zu schwach sind oder im Vergleich zu anderen kardialen Signalen zu schwach oder zu wenig ausgeprägt erscheinen), wird die Position der Elektrode angepasst. Die Schritte des Ausführens einer Stimulation des His-Bündels, der Detektion eines Elektrokardiogramms, der automatischen Identifikation von His-Bündel-spezifischen Signalen im Elektrokardiogramm, der Markierung dieser identifizierten Signale und des Ausgebens des derart markierten Elektrokardiogramms werden dann so oft wiederholt, bis die markierten Signale einem wählbaren Kriterium entsprechen. Bei diesem wählbaren Kriterium kann es sich um das zuvor genannte vordefinierte bzw. vorgebbare Gütekriterium handeln. Beispielsweise kann dieses Kriterium ein qualitatives oder quantitatives Kriterium der detektierten und automatisch identifizierten His-Bündel-spezifischen Signale sein. Dabei kann ein absoluter Wert oder ein relativer Wert der entsprechenden Signale zur Überprüfung, ob das Kriterium erfüllt ist oder nicht, herangezogen werden.

Wenn das wählbare Kriterium erfüllt ist, das heißt, wenn beispielsweise ausreichend große oder ausreichend ausgeprägte oder im Verhältnis zu anderen kardialen Signalen insgesamt ausreichende His-Bündel-spezifische Signale im Elektrokardiogramm identifiziert und markiert wurden, wird die Elektrode an der zuletzt gewählten Position implantiert. Denn dann ist diese Position geeignet, das His-Bündel des Herzens in ausreichender Weise zu stimulieren.

Ein Aspekt der vorliegenden Erfindung betrifft eine implantierbare Anordnung zur Stimulation eines menschlichen oder tierischen Herzens mit den nachfolgend erläuterten Merkmalen. Eine derartige Anordnung weist einen Prozessor, eine Speichereinheit, eine Stimulationseinheit und eine Detektionseinheit auf. Die Stimulationseinheit dient zum Stimulieren des His-Bündels eines menschlichen oder tierischen Herzens. Sie kann für eine derartige Stimulation geeignet oder aber spezifisch dafür vorgesehen und eingerichtet sein. Die Detektionseinheit dient zum Detektieren eines elektrischen Signals desselben Herzens.

Erfindungsgemäß zeichnet sich diese Anordnung dadurch aus, dass die Speichereinheit ein computerlesbares Programm aufweist, das den Prozessor dazu veranlasst, die nachfolgend erläuterten Schritte auszuführen, wenn es auf dem Prozessor ausgeführt wird.

Zunächst wird mittels der Detektionseinheit ermittelt, ob in einem menschlichen oder tierischen Herz, in das die Anordnung implantiert ist, eine Tachykardie vorliegt.

Wenn eine derartige Tachykardie erkannt wurde, wird eine His-Bündel-Stimulation mittels der Stimulationseinheit durchgeführt. Dabei wird die His-Bündel-Stimulation durch Abgabe mindestens eines Stimulationspulses ausgeführt. Dieser Stimulationspuls weist eine Amplitude auf, die in einem Bereich von 7,5 V bis 30 V, insbesondere von 12 V bis 30 V, insbesondere von 15 V bis 25 V, insbesondere von 20 V bis 22 V, insbesondere von mehr als 20 V bis 30 V, insbesondere von mehr als 20 V bis 25 V, insbesondere von mehr als 20 V bis 22 V liegt. Ferner weist der Stimulationspuls eine Pulsbreite auf, die in einem Bereich von 1 ms bis 15 ms, insbesondere von 2 ms bis 12 ms, insbesondere von 3 ms bis 10 ms, insbesondere von 4 ms bis 9 ms, insbesondere von 5 ms bis 8 ms liegt.

Durch einen Stimulationspuls mit einer derartigen Amplitude und einer derartigen Pulsbreite kann eine besonders effektive Stimulation des His-Bündels erreicht werden. Dabei ist es nicht unbedingt erforderlich, dass eine Stimulationselektrode der Stimulationseinheit unmittelbar im His-Bündel angeordnet ist. Vielmehr kann mit einem Stimulationspuls, der eine derartige Stimulationsamplitude und eine derartige Pulsbreite aufweist, eine Stimulation des His-Bündels auch dann erreicht werden, wenn die entsprechende Stimulationselektrode am His-Bündel oder in der Nähe des His-Bündels angeordnet ist. Dadurch wird die Implantation einer solchen Stimulationselektrode vereinfacht. Denn die exakte Erfassung des His-Bündels ist für eine spätere wirksame Stimulation des His-Bündels bei einer derartigen Spezifikation des Stimulationspulses nicht so entscheidend wie in anderen Fällen. Allerdings erleichtert auch bei der gewählten Stimulationsamplitude und der gewählten Pulsbreite des Stimulationspulses eine genaue Erfassung des His-Bündels eine spätere Stimulation des His-Bündels.

In einer Variante veranlasst das Programm den Prozessor dazu, eine detektierte Tachykardie in eine von mindestens zwei Klassen zu klassifizieren. In dieser Variante ist die implantierbare Anordnung folglich in der Lage, zwischen unterschiedlichen tachykarden Rhythmusstörungen des Herzens diskriminieren zu können. Dann lassen sich tachykarde Rhythmusstörungen des Herzens, bei denen eine Stimulation des His-Bündels eine besonders gute Behandlung verspricht, von anderen tachykarden Rhythmusstörungen unterscheiden, bei denen eine Behandlung des His-Bündels weniger erfolgversprechend ist. In einer Variante kann die Auslösung einer Stimulation des His-Bündels davon abhängen, in welche Klasse die detektierte Tachykardie klassifiziert wird.

In einer Variante ist eine erste der mindestens zwei Klassen für eine AV-Knoten-Reentrytachykardie (AVNRT) vorgesehen. Denn bei einer AV-Knoten-Reentrytachykardie ist eine Stimulation des His-Bündels besonders geeignet, um die AV-Knoten-Reentrytachykardie zu beenden und das behandelte Herz wieder in einen normalen Zustand zu überführen. Dabei ist es nicht erforderlich, zusätzlich Medikamente zu geben oder eine Ablationstherapie vorzunehmen. Vielmehr genügt alleine die Stimulation des His-Bündels, um eine detektierte AV-Knoten-Reentrytachykardie zu beenden. Somit bietet sich die erfindungsgemäß beanspruchte Vorrichtung insbesondere dazu an, eine detektierte AV-Knoten-Reentrytachykardie zu behandeln, wobei diese Behandlung nicht die gravierenden Nebenwirkungen und Risiken der aus dem Stand der Technik bekannten Behandlungsmethoden einer AV-Knoten-Reentrytachykardie aufweist.

In einer Variante veranlasst das Programm den Prozessor dazu, die His-Bündel-Stimulation nur dann auszuführen, wenn die Tachykardie als AV-Knoten-Reentrytachykardie identifiziert wurde. In dieser Variante wird die implantierbare Anordnung lediglich zur Behandlung der AV-Knoten-Reentrytachykardie eingesetzt, nicht jedoch zur Behandlung anderer tachykarder Rhythmusstörungen des Herzens bzw. nicht zur Behandlung anderer tachykarder Rhythmusstörungen des Herzens mittels einer His-Bündel-Stimulation.

In einer Variante veranlasst das Programm den Prozessor dazu, die Signale eines Elektrokardiogramms auszuwerten, um zu ermitteln, ob eine Tachykardie vorliegt. Diese Auswertung kann beispielsweise in der Detektionseinheit oder in einer separaten Auswerteeinheit erfolgen. Dabei kann beispielsweise eine morphologische Analyse des Elektrokardiogramms erfolgen. Hierzu eignet sich ein morphologisches Analysekriterium des entsprechenden Elektrokardiogramms. Zur Identifikation dieses morphologischen Analysekriteriums kann beispielsweise eine Mustererkennung oder Bilderkennung in dem Elektrokardiogramm durchgeführt werden, um bestimmte Muster zu identifizieren, die typisch für das Vorliegen einer Tachykardie sind.

In einer Variante handelt es sich bei dem Elektrokardiogramm um ein intrakardiales Elektrogramm oder ein Fernfeld-Elektrokardiogramm (auch unter dem teilweise englischen Fachbegriff Far-Field-Elektrokardiogramm bzw. Far-Field-EKG bekannt). Es ist auch möglich, sowohl ein intrakardiales Elektrogramm als auch ein Fernfeld-EKG auszuwerten.

In einer Variante veranlasst das Programm den Prozessor dazu, zur Ermittlung, ob eine Tachykardie vorliegt, eine zeitliche Abfolge atrialer und ventrikulärer Signale im Elektrokardiogramm auszuwerten. So deuten nahezu gleichzeitig auftretende atriale und ventrikuläre Signale bei einer erhöhten Herzfrequenz auf eine AV-Knoten-Reentrytachykardie hin. In einem solchen Fall kann die detektierte Tachykardie dann als AV-Knoten-Reentrytachykardie klassifiziert werden.

In einer Variante wird die His-Bündel-Stimulation von der Stimulationseinheit mit einem definierten bzw. vorgebbaren Kopplungsintervall an eine intrinsische tachykarde Erregung des zu behandelnden Herzens angekoppelt. Dabei weist das Programm entsprechende Befehle auf, die den Prozessor dazu veranlassen, die Stimulationseinheit entsprechend anzusteuern.

In einer Variante veranlasst das Programm den Prozessor dazu, die His-Bündel-Stimulation als eine Pulsfolge mit mindestens zwei Pulsen auszuführen. Dabei wird in einer Variante ein zeitlicher Abstand zwischen zwei Pulsen dieser Pulsfolge gewählt, der kleiner als die Zykluslänge der ermittelten Tachykardie ist. Das heißt, die His-Bündel-Stimulation erfolgt in dieser Variante als sogenannte Overdrive-Stimulation.

In einer anderen Variante wird zur His-Bündel-Stimulation ebenfalls eine Pulsfolge mit mindestens zwei Pulsen verwendet. Allerdings wird in dieser Variante ein zeitlicher Abstand zwischen zwei Pulsen der Pulsfolge derart gewählt, dass er größer als die Zykluslänge der ermittelten Tachykardie ist. Das heißt, in dieser Variante erfolgt die His-Bündel-Stimulation als sogenannte Underdrive-Stimulation.

Die Entscheidung, ob eine Overdrive-Stimulation oder eine Underdrive-Stimulation ausgeführt wird, kann beispielsweise anhand der spezifischen Ausprägung der ermittelten Tachykardie, insbesondere der AV-Knoten-Reentrytachykardie, getroffen werden.

In einer Variante veranlasst das Programm den Prozessor dazu, einen zeitlichen Abstand zwischen zwei Pulsen einer zur His-Bündel-Stimulation eingesetzten Pulsfolge aus mindestens zwei Pulsen mit einem frei wählbaren Anpassungsfaktor an eine zuvor ermittelte Zykluslänge der detektierten Tachykardie, insbesondere der detektierten AV-Knoten-Reentrytachykardie, anzupassen. Dann erfolgt die His-Bündel-Stimulation mit genau derselben zeitlichen Ausprägung wie die ermittelte Tachykardie; die His-Bündel-Stimulation ist also mit der detektierten Tachykardie synchronisiert.

In einer weiteren Variante veranlasst das Programm den Prozessor dazu, die His-Bündel-Stimulation mittels eines einzelnen Pulses auszuführen. Wenn ein derartiger einzelner Puls eingesetzt wird, kann eine His-Bündel-Stimulation besonders einfach durchgeführt werden, da eine zeitliche Abfolge zweier oder mehrerer Pulse nicht berücksichtigt werden muss. In einer Variante wird ein einzelner Stimulationspuls jedoch an die intrinsische Erregung des zu behandelnden Herzens angekoppelt, also auf die intrinsische Erregung des Herzens abgestimmt.

Ein Aspekt der vorliegenden Erfindung betrifft ein Computerprogrammprodukt mit einem computerlesbaren Code, der einen Prozessor dazu veranlasst, die nachfolgend erläuterten Schritte auszuführen, wenn er auf dem Prozessor ausgeführt wird.

Zunächst wird mittels einer Detektionseinheit erfasst, ob in einem menschlichen oder tierischen Herz eine Tachykardie vorliegt.

Wenn eine derartige Tachykardie detektiert wurde, wird eine His-Bündel-Stimulation mittels einer Stimulationseinheit ausgeführt. Dabei wird mindestens ein Stimulationspuls von der Stimulationseinheit abgegeben. Dieser Stimulationspuls weist dabei eine Amplitude auf, die in einem Bereich von 7,5 V bis 30 V liegt. Der Stimulationspuls weist ferner eine Pulsbreite auf, die im Bereich von 1 ms bis 15 ms liegt.

Ein Aspekt der vorliegenden Erfindung betrifft ein Verfahren zur Behandlung eines menschlichen oder tierischen Patienten, der eine solche Behandlung benötigt. Diese Behandlung erfolgt mittels einer implantierbaren Anordnung zur Stimulation des Herzens des Patienten. Diese Anordnung ist dem Patienten dabei implantiert. Die Anordnung weist einen Prozessor, eine Speichereinheit, eine Stimulationseinheit und eine Detektionseinheit auf. Die Stimulationseinheit dient zum Stimulieren des His-Bündels des Herzens des Patienten. Sie kann für eine derartige Stimulation geeignet oder aber spezifisch dafür vorgesehen und eingerichtet sein. Die Detektionseinheit dient zum Detektieren eines elektrischen Signals des Herzens des Patienten. Das Verfahren weist die nachfolgend erläuterten Schritte auf.

Zunächst wird mittels der Detektionseinheit erfasst, ob in den Herzen des Patienten eine Tachykardie vorliegt.

Wenn eine derartige Tachykardie detektiert wurde, wird eine His-Bündel-Stimulation mittels der Stimulationseinheit ausgeführt. Dabei wird für die His-Bündel-Stimulation von der Stimulationseinheit mindestens ein Stimulationspuls abgegeben. Dieser Stimulationspuls weist eine Amplitude auf, die in einem Bereich von 7,5 V bis 30 V liegt. Darüber hinaus weist der Stimulationspuls eine Pulsbreite auf, die in einem Bereich von 1 ms bis 15 ms liegt.

Sämtliche Varianten und alternativen Ausgestaltungen, die in Zusammenhang mit den verschiedenen implantierbaren Anordnungen beschrieben wurden, sind beliebig miteinander kombinierbar und auf die jeweils anderen Anordnungen übertragbar. Sie sind in analoger Weise auch in beliebiger Kombination auf die beschriebene Analyseeinheit, die beschriebenen Verfahren und die beschriebenen Computerprogrammprodukte übertragbar. Ferner sind die verschiedenen Varianten der Analyseeinheit beliebig miteinander kombinierbar und auf die unterschiedlichen implantierbaren Anordnungen sowie in analoger Weise auch auf die beschriebenen Verfahren und die beschriebenen Computerprogrammprodukte übertragbar. Ferner sind die beschriebenen Varianten der Verfahren beliebig miteinander kombinierbar und auf die jeweils anderen Verfahren sowie auf die Computerprogrammprodukte und die Anordnungen sowie die Analyseeinheit übertragbar. In gleicher Weise sind die beschriebenen Varianten der Computerprogrammprodukte beliebig miteinander kombinierbar und auf die jeweils anderen Computerprogrammprodukte sowie auf die beschriebenen Verfahren und die beschriebenen Anordnungen sowie die Analyseeinheit übertragbar.

Weitere Details von Aspekten der Erfindung werden nachfolgend in Zusammenhang mit Ausführungsbeispielen und Zeichnungen näher erläutert. Es zeigen:
- Figur 1: ein Blockschaltbild eines Ausführungsbeispiels einer implantierbaren Anordnung zur Stimulation des menschlichen oder tierischen Herzens;
- Figur 2: eine schematische Darstellung eines Ausführungsbeispiels einer implantierbaren Anordnung des menschlichen oder tierischen Herzens;
- Figur 3: ein Blockschaltbild eines Ausführungsbeispiels einer implantierbaren Anordnung zur Stimulation des menschlichen oder tierischen Herzens, die im Betrieb eine Stimulationserfolgskontrolle ausführt;
- Figur 4: ein vereinfachtes Blockschaltbild eines Ausführungsbeispiels einer Analyseeinrichtung;
- Figur 5: ein Ausführungsbeispiel einer von der Analyseeinrichtung der Figur 4 ausgegebenen Darstellung; und
- Figur 6: ein Blockschaltbild eines Ausführungsbeispiels einer implantierbaren Anordnung zur Stimulation des menschlichen oder tierischen Herzens.

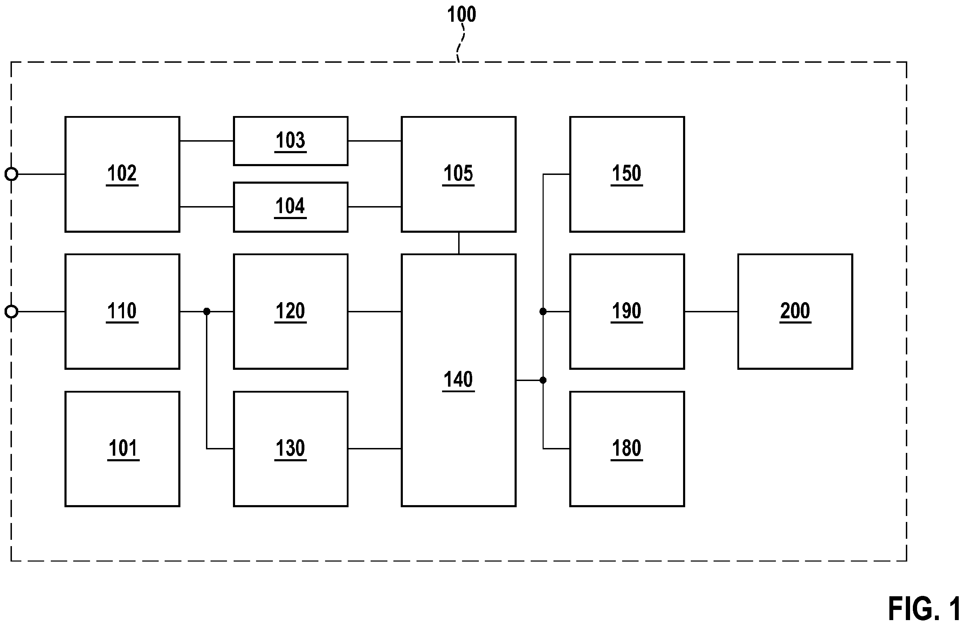

Die Figur 1 zeigt ein Blockschaltbild eines Ausführungsbeispiels eines Herzschrittmachers 100, der als implantierbare Anordnung zur Stimulation des menschlichen oder tierischen Herzens dient. Der Herzschrittmacher 100 weist eine Energiequelle 101 und einen ersten Elektrodenanschluss 102 auf, der als erster Stimulationsausgang dient. Mit dem Elektrodenanschluss 102 verbunden ist eine erste Stimulationseinheit 103, von der Stimulationsimpulse durch den ersten Elektrodenanschluss 102 zu einer ersten Elektrode geleitet werden können. Darüber hinaus ist eine erste Detektionseinheit 104 mit dem ersten Elektrodenanschluss 102 verbunden. Die erste Stimulationseinheit 103 und die erste Detektionseinheit 104 wirken als konventionelle Detektions- und Stimulationsstufe des Herzschrittmachers 100.

Um für eine zeitlich definierte Abgabe von Stimulationsimpulsen durch die erste Stimulationseinheit 103 zu sorgen und die entsprechenden Stimulationsimpulse an die durch die erste Detektionseinheit 104 detektierten Signale eines Herzens anzupassen, weist der Herzschrittmacher 100 ferner einen ersten Zeitgeber 105 auf, der ebenfalls in konventioneller Art und Weise funktioniert.

Darüber hinaus weist der Herzschrittmacher 100 einen zweiten Elektrodenanschluss 110 auf, der als zweiter Stimulationsausgang dient. Er ist mit einer zweiten Stimulationseinheit 120 und einer zweiten Detektionseinheit 130 verbunden. Die zweite Stimulationseinheit ist spezifisch dafür ausgebildet und hergerichtet, eine Stimulation des His-Bündels des zu behandelnden Herzens durchzuführen. Die zweite Detektionseinheit 130 ist spezifisch dafür ausgebildet und hergerichtet, ein elektrisches Signal des His-Bündels dieses Herzens zu detektieren.

Sowohl die zweite Stimulationseinheit 120 als auch die zweite Detektionseinheit 130 sind mit einem zweiten Zeitgeber 140 verbunden. Dieser steht darüber hinaus in Wirkverbindung mit dem ersten Zeitgeber 105. Mittels des zweiten Zeitgebers 140 ist es möglich, die von der zweiten Stimulationseinheit 120 abzugebenden Stimulationsimpulse mit den Stimulationsimpulsen, die von der ersten Stimulationseinheit 103 abgegeben werden, zu synchronisieren. Auf diese Weise kann eine Stimulation des His-Bündels des Herzens zu einem Zeitpunkt erfolgen, an dem des His-Bündels für eine derartige Stimulation besonders empfänglich ist und der sich in physiologisch sinnvoller Art und Weise zur Wiederherstellung eines natürlichen Herzrhythmus anbietet. Dabei eröffnet der zweite Zeitgeber 140 auch die Möglichkeit, über Signale, die von der zweiten Detektionseinheit 130 erfasst werden, eine Synchronisation der zeitlichen Abgabe von Stimulationsimpulsen, die von der ersten Stimulationseinheit 103 abgegeben werden, mit Stimulationsimpulsen, die von der zweiten Stimulationseinheit 120 abgegeben werden, zu erreichen.

Dem zweiten Zeitgeber 140 nachgeordnet sind ein His-Bündel-Markerkanal 150, eine His-Bündel-Reizschwellentesteinheit, die als erste Reizschwellentestvorrichtung dient, und ein His-Bündel-Diagnostikspeicher 190.

Mittels des His-Bündel-Markerkanals 150 ist es möglich, ein Elektrokardiogramm (EKG) oder ein intrakardiales Elektrogramm (IEGM) aus dem Herzschrittmacher 100 auszulesen, wobei das EKG bzw. das IEGM mit Markierungen versehen ist, die spezifisch für eine His-Bündel-Stimulation mittels der zweiten Stimulationseinheit 120 und/oder spezifisch für eine Detektion eines Signals des His-Bündels mittels der zweiten Detektionseinheit 130 sind. Auf diese Weise lassen sich über den His-Bündel-Markerkanal 150 also EKGs oder IEGMs aus dem Herzschrittmacher 100 abrufen, die mit His-Bündel-spezifischer Informationen versehen sind.

Die His-Bündel-Reizschwellentesteinheit 180 dient dazu, eine Reizschwelle des His-Bündels zu bestimmen, bevor eine entsprechende Stimulation des His-Bündels mittels der zweiten Stimulationseinheit 120 durchgeführt wird. Auf diese Weise kann mittels der zweiten Stimulationseinheit 120 stets ein ausreichend starker Stimulationsimpuls bereitgestellt werden, ohne jedoch hierfür mehr Energie als nötig aufwenden zu müssen. Somit dient die His-Bündel-Reizschwellentesteinheit 180 dazu, einerseits sicherzustellen, dass die von der zweiten Stimulationseinheit 120 abgegebenen Stimulationsimpulse stark genug sind, um eine Erregung des His-Bündels zu erreichen, andererseits aber die durch die zweite Stimulationseinheit 120 abgegebenen Stimulationsimpulse nur eine möglichst geringe Energie aufweisen, sodass die Energiequelle 101 des Herzschrittmachers 100 durch die zweite Stimulationseinheit 120 so gering wie möglich belastet wird.

Im His-Bündel-Diagnostikspeicher 190, der beispielsweise als Speicherbereich einer größeren Speichereinheit ausgestaltet sein kann, werden Ereignisse gespeichert, die die His-Bündel-Stimulation bzw. die His-Bündel-Aktivität betreffen. So dient der His-Bündel-Diagnostikspeicher dazu, von der zweiten Stimulationseinheit 120 abgegebenen Stimulationsimpulsen aufzuzeichnen und darüber hinaus auch Daten aufzuzeichnen, die von der zweite Detektionseinheit 130 in Bezug auf das His-Bündel des zu behandelnden Herzens detektiert wird.

Dem His-Bündel-Diagnostikspeicher 190 ist eine His-Bündel-Fernüberwachungs- und - programmiereinheit 200 zugeordnet, über die sich der His-Bündel-Diagnostikspeicher 190 auslesen lässt. Darüber hinaus können mittels der His-Bündel-Fernüberwachungs- und - programmiereinheit 200 die zweite Stimulationseinheit 120 und/oder die zweite Detektionseinheit 130 überwachen. Ferner ist es möglich, mittels des His-Bündel-Fernüberwachungs- und -programmiereinheit 200 eine Anpassung der von der zweiten Stimulationseinheit 120 abzugebenden His-Bündel-Stimulation vorzunehmen. Somit gestattet diese His-Bündel-Fernüberwachungs- und -programmiereinheit 200 einen Zugriff auf spezifische Komponenten des Herzschrittmachers 100, wobei sowohl ein Auslesen von Daten als auch ein Schreiben von Daten ermöglicht wird.

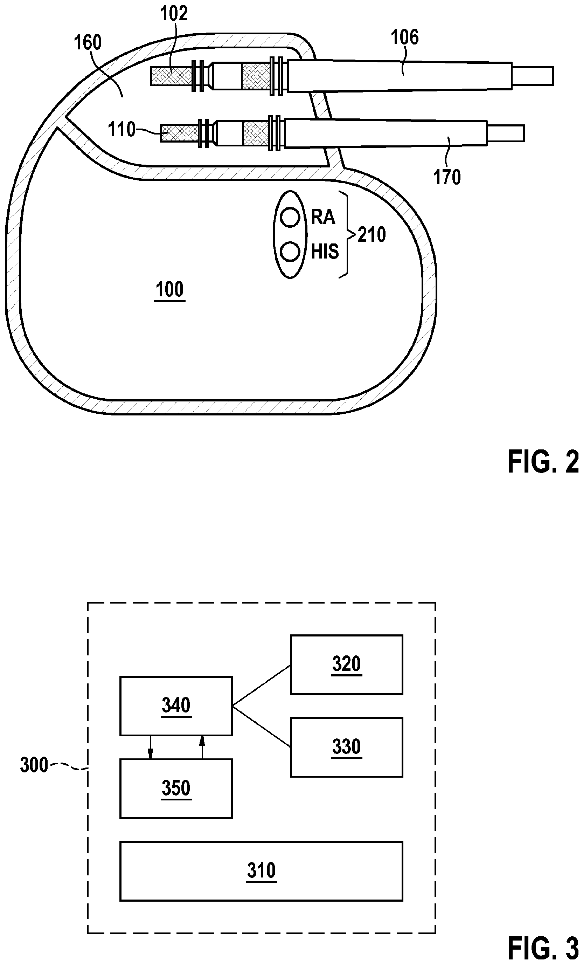

Die Figur 2 zeigt eine schematische Seitenansicht des Herzschrittmachers 100, dessen Blockschaltbild in der Figur 1 dargestellt ist. Dabei werden gleiche Elemente mit den gleichen Bezugszeichen versehen.

In der schematischen Darstellung der Figur 2 ist ein Header 160 zu sehen, in dem der erste Elektrodenanschluss 102 und der zweite Elektrodenanschluss 110 ausgebildet sind. Dabei ist eine erste Elektrode 106 in den ersten Elektrodenanschluss 102 und eine zweite Elektrode 170 in den zweiten Elektrodenanschluss 110 eingesteckt. Die erste Elektrode 106 und die zweite Elektrode 107 sind in der Darstellung der Figur 2 ausschnittsweise dargestellt.

Die erste Elektrode 106 dient einer konventionellen Stimulation eines beliebigen Herzareals einer der Herzkammern. Sie dienen gleichermaßen einer Detektion elektrischer Signale in einer dieser Herzkammern.

Die zweite Elektrode 170 dient spezifisch einer His-Bündel-Stimulation sowie einer Detektion von His-Bündel-spezifischen elektrischen Signalen. Um einem Anwender eine leichtere Anwendung des Herzschrittmachers 100 zu ermöglichen, ist die zweite Elektrode 170 durch die Aufschrift "HIS" als His-Bündel-Elektrode gekennzeichnet. Damit diese zweite Elektrode 170 in den richtigen Elektrodenanschluss, nämlich den zweiten Elektrodenanschluss 110, eingesteckt wird, ist dieser mit der Zusatzkennzeichnung "HIS" im Header 160 des Herzschrittmachers 100 versehen.

Dabei kann auch vorgesehen sein, den zweiten Elektrodenanschluss 110 strukturell anders als den ersten Elektrodenanschluss 102 auszugestalten, sodass ein Einstecken der zweiten Elektrode 170 (also der His-Bündel-Elektrode) in den ersten Elektrodenanschluss 102 gar nicht möglich ist.

Neben einer Aufschrift wie "HIS" kann darüber hinaus eine farbliche Kodierung der zweiten Elektrode 170 und/oder des zweiten Elektrodenanschlusses 110 bzw. eines entsprechenden Bereich des Headers 160 vorgesehen sein.

Der Herzschrittmacher 100 weist darüber hinaus an seiner Seite eine Markierung 210 auf, die den ersten Elektrodenanschluss 100 und den zweiten Elektrodenanschluss 110 schematisch darstellt und mit einer entsprechenden Kennzeichnung versieht. Dabei weist die Markierung 210 darauf hin, dass der erste (obere) Elektrodenanschluss 102 zum Anschluss einer in das rechte Atrium (RA) führenden ersten Elektrode 106 und der zweite (untere) Elektrodenanschluss 110 zum Anschluss einer zum His-Bündel führenden zweiten Elektrode 170 vorgesehen ist. Diese zusätzliche Markierung 210 erleichtert einem Anwender des Herzschrittmachers 100 zusätzlich das Anschließen der korrekten Elektroden 106, 170 an den dafür vorgesehenen Elektrodenanschlüssen 102, 110.

Die Figur 3 zeigt ein Blockschaltbild eines Ausführungsbeispiels eines Herzschrittmachers 300, der als implantierbare Anordnung zur Stimulation des menschlichen oder tierischen Herzens dient. Dieser Herzschrittmacher 300 führt eine Stimulationserfolgskontrolle durch und ist in der Lage, einen internen Steuerungsparameter in Abhängigkeit der durchgeführten Stimulationserfolgskontrolle automatisch anzupassen.

Der Herzschrittmacher weist eine Energiequelle 310 und eine His-Bündel-Stimulationseinheit 320 auf, die als Stimulationseinheit dient. Ferner ist eine Detektionseinheit 330 vorgesehen, die als Detektionseinheit dient. Die His-Bündel-Stimulationseinheit 320 und die Detektionseinheit 330 stehen in Wirkverbindung mit einem Prozessor 340. Dieser Prozessor 340 wiederum kann auf eine Speichereinheit 350 zugreifen und Daten aus dieser Speichereinheit 350 empfangen bzw. zu ihr senden.

In der Speichereinheit 350 ist ein Programm hinterlegt, mit dem der Prozessor 340 bestimmte Schritte durchführen kann. So veranlasst der Prozessor 340 die His-Bündel-Stimulationseinheit 320 dazu, mittels einer der Figur 3 nicht dargestellten Stimulationselektrode eine kardiale Stimulation (insbesondere des His-Bündels) durchzuführen. Anschließend wird die Detektionseinheit 330 vom Prozessor 340 dazu veranlasst, ein kardiales elektrisches Signal aus dem zuvor stimulierten Herzen zu erfassen. Dieses Signal wird dann verwendet, um einen Erregungszustand des Herzens zu ermitteln. Der Erregungszustand wird in eine von mindestens drei unterschiedlichen Klassen klassifiziert. Anschließend erfolgt in Abhängigkeit der durchgeführten Klassifikation eine automatische Anpassung eines spezifischen Steuerparameters des Herzschrittmachers 300.

Die Schritte der Ermittlung des Erregungszustands, der Klassifikation des Erregungszustands und der automatischen Anpassung mindestens eines Steuerparameters können in einem Stimulationserfolgskontrollmodul durchgeführt werden, das entweder hardwaretechnisch oder softwaretechnisch realisiert sein kann. Im Ausführungsbeispiel der Figur 3 ist dieses Stimulationserfolgskontrollmodul softwaretechnisch realisiert, sodass es nicht gesondert dargestellt ist.

Durch die automatische Anpassung mindestens eines Steuerparameters des Herzschrittmachers 300 kann auf besonders einfache Weise eine Therapieerfolgskontrolle einer zuvor durchgeführten His-Bündel-Stimulation erfolgen, wobei durch die automatische Anpassung eines Steuerparameters eine besonders sichere und wirksame Therapie auch über einen langen Zeitraum gewährleistet werden kann.

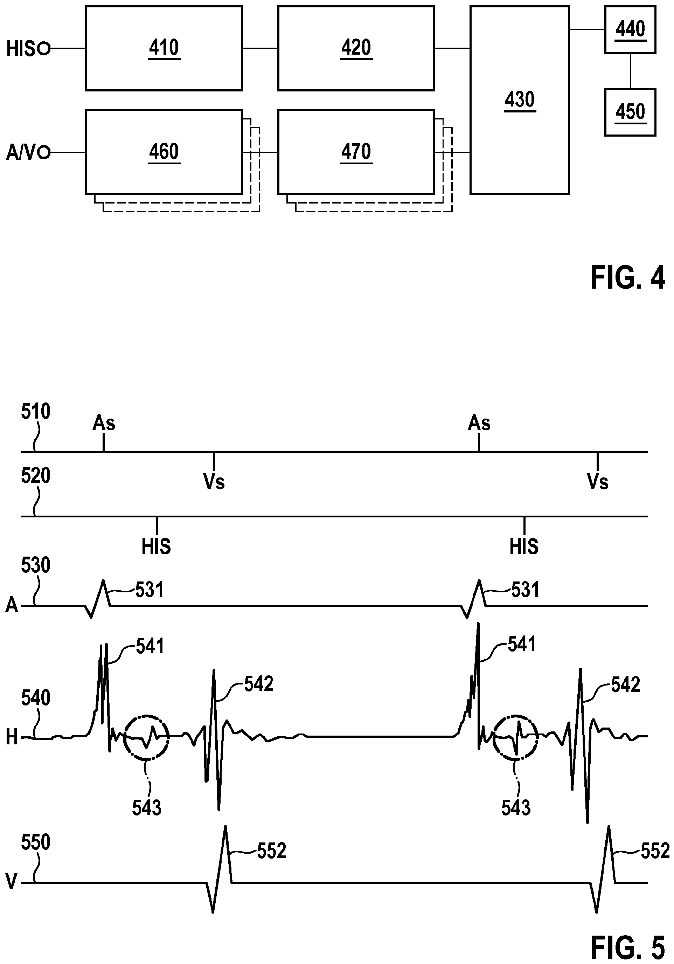

Die Figur 4 zeigt ein vereinfachtes Blockschaltbild eines His-Bündel-Pace-Sense-Analysators (His-PSA), der als Analyseeinrichtung dient. Dieser His-PSA weist eine His-Bündel-Stimulationseinheit 410 auf, die eine Elektrodenschnittstelle umfasst, mittels der eine His-Bündel-Elektrode an die His-Bündel-Stimulationseinheit 410 angeschlossen werden kann. Die His-Bündel-Stimulationseinheit 410 dient zur Abgabe einer hochenergetischen Stimulation, mit der das His-Bündel eines menschlichen oder tierischen Herzens gut stimuliert werden kann.

Der His-PSA weist darüber hinaus eine erste Detektionseinheit 420 auf, die ebenfalls direkt auf eine Elektrode zugreifen kann, mit der eine Stimulation des His-Bündels erfasst werden kann. Die erste Detektionseinheit 420 ist dabei dazu vorgesehen und eingerichtet, ein Elektrokardiogramm in Form eines breitbandigen intrakardialen Elektrogramms (IEGM) aufzuzeichnen und einer morphologischen Signalanalyse zu unterziehen. Mithilfe eines digitalen Signalprozessors (DSP), der Bestandteil der ersten Detektionsvorrichtung 420 ist, können His-Bündel-typische Signalmorphologien innerhalb des erfassten Elektrokardiogramms identifiziert und markiert werden. Die erste Detektionseinheit 420 gibt das derart weiterverarbeitete IEGM dann an eine Steuer- und Anzeigeeinheit 430 weiter, die als Ausgabegerät zur Ausgabe des markierten IEGMs dient.

Die Steuer-und Ausgabeeinheit 430 ist darüber hinaus mit einem Prozessor 440 verbunden, welcher wiederum auf eine Speichereinheit 450 zugreifen kann. Auf diese Weise ist es möglich, Programminformationen aus der Speichereinheit 450 durch den Prozessor 440 abzurufen und über die Steuer- und Anzeigeeinheit 430 an die His-Bündel-Stimulationseinheit 410 und die erste Detektionseinheit 420 zu übermitteln.

Darüber hinaus weist der His-PSA eine oder mehrere weitere Stimulationseinheiten 460 auf, die als konventionelle Stimulationseinheiten ausgestaltet sind und konventionelle Elektrodenschnittstellen zum Anschluss atrialer oder ventrikulärer Elektroden umfassen. Dabei können konventionelle Stimulationsspezifikationen der entsprechenden Elektroden vorgesehen sein. Neben den weiteren Stimulationseinheiten 460 sind zudem eine oder mehrere weitere Detektionseinheiten 470 vorgesehen, die der Detektion und Auswertung atrialer und ventrikulärer kardialer Signale dienen. Hierfür verwenden diese weiteren Detektionseinheiten 470 konventionelle Detektionsspezifikationen. Mittels dieser weiteren Detektionseinheiten 470 ist es möglich, ventrikuläre und atrialer Signale in aufgenommenen Elektrokardiogrammen zu identifizieren und zu markieren. Mithin kann die Steuer- und Anzeigeeinheit 430 sowohl His-Bündel-spezifische Signale als auch atriale und/oder ventrikuläre Signale in einem Elektrokardiogramm wie einem IEGM darstellen. Dabei ist die Steuer- und Anzeigeeinheit 430 in der Lage, unterschiedliche Kanäle (insbesondere einen atrialen Kanal, einen ventrikulären Kanal und einen His-Bündel-spezifischen Kanal) gesondert voneinander darzustellen.

Dies ist in der Figur 5 beispielhaft schematisch dargestellt. So zeigt die Figur 5 von oben nach unten einen ersten Markerkanal 510 zur Markierung atrialer Signale As und ventrikuläre Signale Vs.

Darunter ist ein zweiter Markerkanal 520 dargestellt, der der Markierung von His-Bündel-spezifischen Signalen HIS dient.

In einem atrialen IEGM-Kanal 530 werden in schematischer Weise nur atriale Signale 531 dargestellt.

In einem His-Bündel-spezifischen Kanal 540 sind neben atrialen Signalen 541 und ventrikulären Signalen 542 His-Bündel-spezifische Signale 543 dargestellt und gesondert markiert. Dies kann typischerweise in farblicher Hervorhebung erfolgen. In der Darstellung der Figur 5 sind die His-Bündel-spezifischen Signale 543 eingekreist dargestellt.

In einem ventrikulären IEGM-Kanal 550 sind schließlich in schematischer Darstellung lediglich ventrikuläre Signale 552 dargestellt.

Ein Anwender kann sich mehrere Kanäle 510-550 gleichzeitig oder aber einzelne Kanäle 510-550 getrennt von anderen Kanälen 510-550 anzeigen lassen. So lassen sich die für die spezifische Fragestellung relevanten Informationen für den Anwender besonders einfach filtern. Durch die automatische Identifikation und gesonderte Markierung His-Bündel-spezifische Signale 543 ist es besonders einfach, eine korrekte Positionierung einer His-Bündel-spezifischen Stimulationselektrode zu überprüfen und im Hinblick auf eine möglichst gute Kontaktierung des His-Bündels zu optimieren.

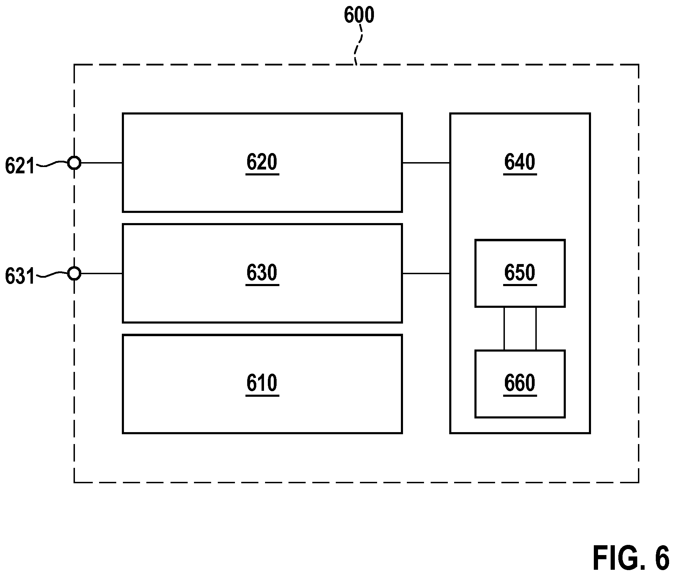

Die Figur 6 zeigt einen Herzschrittmacher 600, die als implantierbare Anordnung zur Stimulation des menschlichen oder tierischen Herzens dient. Dieser Herzschrittmacher 600 ist spezifisch dafür vorgesehen und eingerichtet, eine AV-Knoten-Reentrytachykardie (AVNRT) zu behandeln.

Der Herzschrittmacher 600 umfasst eine Energiequelle 610, eine Tachykardieerkennungs- und Klassifizierungseinheit 620, die als Detektionseinheit dient, und eine His-Bündel-Stimulationseinheit 630, die als Stimulationseinheit dient. Die Tachykardieerkennungs- und Klassifizierungseinheit 620 und die Stimulationseinheit 630 sind mit einer Steuereinheit 640 verbunden. Die Steuereinheit 640 wiederum steht mit einem Prozessor 650 in Wirkverbindung, der auf eine Speichereinheit 660 zugreifen kann.

Die Tachykardieerkennungs- und Klassifizierungseinheit 620 weist einen Elektrodenanschluss 621 auf, an den eine erste Elektrode angeschlossen werden kann. Mittels dieser ersten Elektrode kann die Tachykardieerkennungs- und Klassifizierungseinheit 620 erkennen, ob in dem Herzen des Patienten, der den Herzschrittmacher 600 trägt, eine Tachykardie vorliegt. Die Tachykardieerkennungs- und Klassifizierungseinheit 620 ist darüber hinaus in der Lage, die Art der Tachykardie zu erkennen und zu klassifizieren. So kann insbesondere eine AV-Knoten-Reentrytachykardie mittels der Tachykardieerkennungs- und Klassifizierungseinheit 620 von anderen Tachykardien unterschieden werden.

Wenn eine derartige AV-Knoten-Reentrytachykardie von der Tachykardieerkennungs- und Klassifizierungseinheit 620 erkannt wird, sorgt die Steuereinheit 640 dafür, dass die Stimulationseinheit 630 eine spezifisch für eine Stimulation des His-Bündels des Herzens des Patienten geeignete Stimulation abgibt. Zu diesem Zweck weist die Stimulationseinheit 630 einen Elektrodenanschluss 631 auf, an den im Betrieb des Herzschrittmachers 600 eine zweite Elektrode angeschlossen ist, die im His-Bündel oder derart dicht am His-Bündel angeordnet ist, dass mittels dieser Elektrode eine His-Bündel-Stimulation möglich ist. Das entsprechende Signal gibt die Steuereinheit 640 an die Stimulationseinheit 630, nachdem sie einen entsprechenden Befehl seitens des Prozessors 650 erhalten hat. Auf dem Prozessor 650 läuft ein Programm, das der Prozessor 650 aus der Speichereinheit 660 bezieht.

Die einzelnen Komponenten des Herzschrittmachers 600 werden durch die Energiequelle 610 mit der für den Betrieb erforderliche Energie versorgt.

Die Tachykardieerkennungs- und Klassifizierungseinheit 620 verwendet EKG-Signale, die ihr über den Elektrodenanschluss 621 zugeleitet werden, zur Erkennung und Klassifikation einer Tachykardie, insbesondere zur Erkennung einer AV-Knoten-Reentrytachykardie. Die EKG-Signale können entweder aus dem Atrium und/oder dem Ventrikel des Herzens des Patienten abgeleitet werden. Darüber hinaus ist eine direkte Bereitstellung entsprechender EKG-Signale über eine His-Bündel-Elektrode möglich, beispielsweise über die zweite Elektrode, die auch mit dem zweiten Elektrodenanschluss 631 der Stimulationseinheit 630 verbunden ist.

Zur Klassifikation der festgestellten Tachykardie, also zur Unterscheidung unterschiedlicher Tachykardien voneinander, und insbesondere zur Identifikation einer AV-Knoten-Reentrytachykardie kann die Tachykardieerkennungs- und Klassifizierungseinheit 620 auf morphologische Analysekriterien innerhalb des bereitgestellten Elektrokardiogramms zurückgreifen oder statt einer derartigen morphologischen Analyse des Elektrokardiogramms eine zeitliche Abfolge atrialer und/oder ventrikulärer Signale im Elektrokardiogramm auswerten. Dabei ist es auch möglich, sowohl eine morphologische Analyse eines bereitgestellten Elektrokardiogramms vorzunehmen als auch eine Analyse der zeitlichen Abfolge der in diesem Elektrokardiogramm enthaltenen Signale durchzuführen.

Durch diesen Herzschrittmacher 600 wird eine neue, gerätebasierte Therapie für Tachykardien, insbesondere für die AV-Knoten-Reentrytachykardie, bereitgestellt. Damit wird das Therapiespektrum aktiver Implantate erweitert.

## Patentansprüche

1. Implantierbare Anordnung zur Stimulation eines menschlichen oder tierischen Herzens, aufweisend eine erste Stimulationseinheit (103) und eine erste Detektionseinheit (104), wobei die erste Stimulationseinheit (103) einer Stimulation mindestens einer Herzregion eines menschlichen oder tierischen Herzens dient und wobei die erste Detektionseinheit (104) einer Detektion eines elektrischen Signals mindestens einer Herzregion desselben menschlichen oder tierischen Herzens dient,
**dadurch gekennzeichnet,**
**dass** die Anordnung eine zweite Stimulationseinheit (120) aufweist, die spezifisch dafür ausgebildet und hergerichtet ist, eine Stimulation eines His-Bündels desselben menschlichen oder tierischen Herzens durchzuführen, wobei die zweite Stimulationseinheit (120) für ihre spezifische Ausbildung und Herrichtung eine maximale Stimulationsenergie aufweist, die mindestens 10 % höher als die maximale Stimulationsenergie der ersten Stimulationseinheit (103) ist.

2. Implantierbare Anordnung nach Anspruch 1, **dadurch gekennzeichnet, dass** die Anordnung eine zweite Detektionseinheit (130) aufweist, die spezifisch dafür ausgebildet und hergerichtet ist, ein elektrisches Signal des His-Bündels desselben menschlichen oder tierischen Herzens zu detektieren, wobei die zweite Detektionseinheit (130) für ihre spezifische Ausbildung und Herrichtung mindestens eines der nachfolgenden Merkmale aufweist: a) eine um mindestens 10 % höhere Sensitivität als die Sensitivität der ersten Detektionseinheit (104); b) eine um mindestens 10 % größere Detektionsbandbreite als die Detektionsbandbreite der ersten Detektionseinheit (104); c) eine um mindestens 10 % höhere Abtastrate als die Abtastrate der ersten Detektionseinheit (104).

3. Implantierbare Anordnung nach Anspruch 1 oder 2, **dadurch gekennzeichnet, dass** die Anordnung einen ersten Zeitgeber (105) aufweist, der einer zeitlich definierten Abgabe von Stimulationsimpulsen durch die erste Stimulationseinheit (103) dient, und dass die Anordnung einen zweiten Zeitgeber (140) aufweist, der dafür vorgesehen und eingerichtet ist, einen Zeitpunkt der Abgabe mindestens eines von der zweiten Stimulationseinheit (120) abzugebenden Impuls mit einem Zeitpunkt der Abgabe mindestens eines von der ersten Stimulationseinheit (103) abzugebenden Impuls abzustimmen.

4. Implantierbare Anordnung nach Anspruch 3, **dadurch gekennzeichnet, dass** der zweite Zeitgeber (140) auch dafür dient, einen Zeitpunkt der Abgabe mindestens eines von der ersten Stimulationseinheit (103) abzugebenden Impuls mit einem Zeitpunkt der Abgabe mindestens eines von der zweiten Stimulationseinheit (120) abzugebenden Impuls abzustimmen.

5. Implantierbare Anordnung nach einem der vorherigen Ansprüche, **dadurch gekennzeichnet, dass** die Anordnung eine erste Reizschwellentestvorrichtung (180) aufweist, mit der eine Reizschwelle des His-Bündels eines menschlichen oder tierischen Herzens, das durch die Vorrichtung stimuliert werden soll, ermittelt werden kann, wobei eine Stimulationsenergie der zweiten Stimulationseinheit (120) in Abhängigkeit der ermittelten Reizschwelle eingestellt werden kann.

6. Implantierbare Anordnung nach einem der Ansprüche 2 bis 5, **dadurch gekennzeichnet, dass** die Anordnung eine Speichereinheit aufweist, die einen Speicherbereich (190) umfasst, der ausschließlich einer Speicherung von Daten, die von der zweiten Detektionseinheit (130) erfasst wurden, und/oder von Daten, die eine von der zweiten Stimulationseinheit (120) abgegebenen Stimulation betreffen, dient.

7. Implantierbare Anordnung nach einem der Ansprüche 2 bis 6, **dadurch gekennzeichnet, dass** die Anordnung eine Fernkommunikationseinheit (200) aufweist, die einer Übertragung von Daten, die von der zweiten Detektionseinheit (130) erfasst wurden, und/oder von Daten, die eine Aktivität der zweiten Stimulationseinheit (120) betreffen, und/oder einer Überwachung der zweiten Detektionseinheit (130) und/oder der zweiten Stimulationseinheit (120) und/oder einer Anpassung einer von der zweiten Stimulationseinheit (120) abzugebenden Stimulation dient.

8. Implantierbare Anordnung nach einem der Ansprüche 2 bis 7, **dadurch gekennzeichnet, dass** die Anordnung einen Prozessor und eine Speichereinheit aufweist, wobei die Speichereinheit ein computerlesbares Programm aufweist, das den Prozessor dazu veranlasst, die folgenden Schritte auszuführen, wenn es auf dem Prozessor ausgeführt wird: a) Versetzen der Anordnung in einen His-Bündel-Stimulationsmodus, in dem nur mittels der zweiten Stimulationseinheit (120) eine His-Bündel-Stimulation durchgeführt werden kann; b) Abgeben einer His-Bündel-Stimulation mittels der zweiten Stimulationseinheit (120), wenn mittels der zweiten Detektionseinheit (130) ein die Abgabe rechtfertigendes Ereignis detektiert wurde.

9. Implantierbare Anordnung nach einem der Ansprüche 2 bis 8, **dadurch gekennzeichnet, dass** die Anordnung einen Prozessor und eine Speichereinheit aufweist, wobei die Speichereinheit ein computerlesbares Programm aufweist, das den Prozessor dazu veranlasst, die folgenden Schritte auszuführen, wenn es auf dem Prozessor ausgeführt wird: a) Versetzen der Anordnung in einen Sicherheitsmodus, in dem mindestens eine Stimulation mindestens einer Herzkammer mittels der ersten Stimulationseinheit (103) oder eine His-Bündel-Stimulation mittels der zweiten Stimulationseinheit (120) durchgeführt werden kann; b) Abgeben einer Herzkammer-Stimulation mittels der ersten Stimulationseinheit (103) und/oder Abgeben einer His-Bündel-Stimulation mittels der zweiten Stimulationseinheit (120), wenn mittels der ersten Detektionseinheit (104) und/oder der zweiten Detektionseinheit (130) ein die Abgabe rechtfertigendes Ereignis detektiert wurde.

10. Implantierbare Anordnung nach einem der Ansprüche 2 bis 9, **dadurch gekennzeichnet, dass** die Anordnung einen Markerkanal (150) aufweist, über den ein Elektrokardiogramm oder ein intrakardiales Elektrogramm ausgelesen werden kann, wobei das Elektrokardiogramm oder das intrakardiale Elektrogramm mindestens eine Markierung aufweist, die spezifisch für eine His-Bündel-Stimulation mittels der zweiten Stimulationseinheit (120) und/oder spezifisch für eine Detektion eines Signals des His-Bündels mittels der zweiten Detektionseinheit (130) ist.

11. Computerprogrammprodukt mit einem computerlesbaren Code, der einen Prozessor dazu veranlasst, die folgenden Schritte auszuführen, wenn er auf dem Prozessor ausgeführt wird:
a) Erfassen mittels einer ersten Detektionseinheit (104) und/oder einer zweiten Detektionseinheit (130), ob in einem menschlichen oder tierischen Herz eine zu behandelnde Herzrhythmusstörung vorliegt, wobei die erste Detektionseinheit (104) zur Detektion eines elektrischen Signals mindestens einer Herzregion des menschlichen oder tierischen Herzens vorgesehen ist und die zweite Detektionseinheit (130) spezifisch dafür ausgebildet und hergerichtet ist, ein elektrisches Signal des His-Bündels desselben menschlichen oder tierischen Herzens zu detektieren,
b) wenn eine zu behandelnde Herzrhythmusstörung vorliegt, Ausführen einer atrialen oder ventrikulären Stimulation mittels einer ersten Stimulationseinheit (103) und/oder Ausführen einer His-Bündel-Stimulation mittels einer zweiten Stimulationseinheit (120).

12. Verfahren zur Behandlung eines menschlichen oder tierischen Patienten, der eine solche Behandlung benötigt, mittels einer implantierbaren Anordnung (100) nach einem der Ansprüche 1 bis 10, wobei die Anordnung eine erste Stimulationseinheit (103) und eine erste Detektionseinheit (104) aufweist, wobei die erste Stimulationseinheit (103) einer Stimulation mindestens einer Herzregion des Herzens des Patienten dient und wobei die erste Detektionseinheit (104) einer Detektion eines elektrischen Signals mindestens einer Herzregion des Herzens dient, wobei die Anordnung (100) eine zweite Stimulationseinheit (120) aufweist, die spezifisch dafür ausgebildet und hergerichtet ist, eine Stimulation eines His-Bündels des Herzens des Patienten durchzuführen, wobei die zweite Stimulationseinheit (120) für ihre spezifische Ausbildung und Herrichtung eine maximale Stimulationsenergie aufweist, die mindestens 10 % höher als die maximale Stimulationsenergie der ersten Stimulationseinheit (103) ist, wobei das Verfahren die folgenden Schritte umfasst:
a) Detektieren mittels der ersten Detektionseinheit (104) und/oder einer optional vorgesehenen zweiten Detektionseinheit (130), die spezifisch dafür ausgebildet und hergerichtet ist, ein elektrisches Signal des His-Bündels des Herzens des Patienten zu detektieren, ob ein behandlungspflichtiges Ereignis im Herzen des Patienten erfolgt ist,
b) Durchführen einer Stimulation einer Herzregion des Patienten mittels der ersten Stimulationseinheit (103) und/oder Durchführen einer Stimulation des His-Bündels des Herzens des Patienten mittels der zweiten Stimulationseinheit (120).
